# EUROPÄISCHE PATENTSCHRIFT

(11) **EP 2 960 235 B1**
(45) Veröffentlichungstag und Bekanntmachung des Hinweises auf die Patenterteilung: **26.09.2018**
(21) Anmeldenummer: 14173852.6
(22) Anmeldetag: 25.06.2014
(51) Int. Cl.: C07D 307/12, C07D 307/24, A61K 8/49, C11B 9/00, C11D 3/20, C11D 3/50

(54) **TETRAHYDROFURANDERIVATE ALS RIECHSTOFFE**
TETRAHYDROFURAN DERIVATIVES AS FRAGRANCES
DERIVÉS DE TETRAHYDROFURAN COMME PARFUMS

(43) Veröffentlichungstag der Anmeldung: 30.12.2015
(73) Patentinhaber: Symrise AG, 37603 Holzminden (DE)
(72) Erfinder: Hölscher, Bernd, 37620 Halle (DE); Mansfeld, Marc, 37647 Brevörde (DE); Diaz Gomez, Edison, 38642 Goslar (DE)
(74) Vertreter: Fabry, Bernd

(56) Entgegenhaltungen:
- EP-A1- 1 318 148
- US-A- 2 843 607
- TAKEOKA GARY R ET AL: "Odor thresholds of cyclic esters", OLFACTION AND TASTE XI : PROCEEDINGS OF THE 11TH INTERNATIONAL SYMPOSIUM ON OLFACTION AND TASTE AND OF THE 27TH JAPANESE SYMPOSIUM ON TASTE AND SMELL ; JOINT MEETING HELD AT KOSEI-NENKIN KAIKAN, S1APPORO, JAPAN, JULY 12 - 16, 1993, TOKYO : SPRINGER, 1. Januar 1994 (1994-01-01), Seiten 271-273, XP009165832, ISBN: 3-540-70142-7
- FUJIMA Y ET AL: "A scalable chemoenzymatic preparation of (R)-tetrahydrofuran-2-carboxylic acid", TETRAHEDRON ASYMMETRY, PERGAMON PRESS LTD, OXFORD, GB, Bd. 14, Nr. 10, 16. Mai 2003 (2003-05-16), Seiten 1385-1391, XP004439175, ISSN: 0957-4166, DOI: 10.1016/S0957-4166(03)00249-0
- M. R. SKURKO ET AL: "Proton-acceptor properties of carbonyl-containing derivatives of 2-tetrahydrofuroic acid", BULLETIN OF THE ACADEMY OF SCIENCES OF THE USSR; DIVISION OF CHEMICAL SCIENCES, Bd. 31, Nr. 9, 1. September 1982 (1982-09-01), Seiten 1796-1798, XP055138399, ISSN: 0568-5230, DOI: 10.1007/BF00952379

## Beschreibung

### GEBIET DER ERFINDUNG

Die Erfindung befindet sich auf dem Gebiet der Riech- und Aromastoffe und betrifft primär Riechstoff- und Aromastoffmischungen umfassend spezielle Tetrahydrofuranderivate sowie deren Verwendung in entsprechenden parfümierten Produkten.

### STAND DER TECHNIK

In der Parfümindustrie besteht generell ein ständiger Bedarf an neuen Riechstoffen, da den Konsumenten laufend neue und moderne Düfte mit frischen Duftnoten zur Verfügung gestellt werden sollen. Neue Riechstoffe mit süßlicher, frischer und natürlichen Duftnoten werden in großer Menge und ungezählten Variationen in Parfüms, Riechstoffmischungen (Parfümkompositionen) und Parfümierungen für die verschiedensten Anwendungsgebiete eingesetzt. Wegen der steigenden Nachfrage der Verbraucher nach neuen süßlicher, frischer und natürlichen Duftnoten, besteht in der Parfümindustrie ein ständiger Bedarf an Duftstoffen, mit denen sich in Parfüms neuartige Effekte erzielen und auf diese Art neue Modetrends kreieren lassen. Verbindungen mit süßlicher, frischer und natürlichen Duftnoten sind seit jeher wichtige und begehrte Komponenten in der Duftstoffindustrie, insbesondere in Parfümkompositionen.

Trotz einer Vielzahl bereits vorhandener Riechstoffe besteht in der Parfümindustrie für Kreation neuartiger moderner Parfümkompositionen ein ständiger Bedarf an neuen Riechstoffen mit besonderen geruchlichen Eigenschaften, die geeignet sind, als Grundlage für die Komposition von neuartigen modernen Parfüms mit komplexem Charakter zu dienen. Insbesondere ist der Fokus auf neue Duftstoffe gerichtet, die über Ihre primären, nämlich geruchlichen, Eigenschaften hinaus, zusätzliche positive Sekundäreigenschaften besitzen, wie z.B. eine höhere Stabilität unter bestimmten Anwendungsbedingungen, eine hohe Ausgiebigkeit, eine große Strahlungskraft, gute Diffusivität (d.h. eine gute Raumwirkung), Fülle, Kraft und/oder Natürlichkeit, geruchsverstärkende Eigenschaften oder aber auch bessere dermatologische Eigenschaften gegenüber Riechstoffen mit vergleichbaren primären geruchlichen Eigenschaften.

Neue Duftstoffe, die insbesondere in eine bestimmte Richtung gehen sollen, sind stets schwer zu identifizieren, da zum einen der Mechanismen der Geruchswahrnehmung nicht ausreichend bekannt ist, und auch die Zusammenhänge zwischen der speziellen Geruchswahrnehmung einerseits und der chemischen Struktur des zugehörigen Riechstoffs andererseits nicht hinreichend erforscht sind, so dass häufig bereits geringfügige Änderungen am strukturellen Aufbau eines bekannten Riechstoffs zu starken Änderungen der sensorischen Eigenschaften und Beeinträchtigungen der Verträglichkeit für den menschlichen Organismus, bewirken. Für die Verwendung einer Substanz als Riechstoff sind daher neben einem interessanten Geruchsprofil auch noch andere Eigenschaften des Stoffes wie z.B. die Stabilität, die Kompatibilität mit anderen Riechstoffen, die Löslichkeit, sowie die toxikologische Unbedenklichkeit, Bioabbarbaukeit und Bioakumulation wichtig.

In diesem Zusammenhang sei auf folgende Druckschriften verwiesen:

Gegenstand der US 2843607 (SERVIGNE MARCEL) ist ein Verfahren zur Herstellung von Estern, insbesondere Estern der Tetrahydrofurancarbonsäuren. In den Beispielen IV und V wird die Herstellung von Tetrahydrofuran-2-carbonsäureethylester und Terahydrofuran-2-carbonsäurepropylester ofenbart.

Aus **SPRINGER, S. 271-273, (ISBN: 3-540-70142-7)** ist ein Tetrahydrofuran-2-carbonsäureethylester bekannt, Allerdings geht es in der Veröffentlichung lediglich um die Bestimmung von Geruchsschwellenwerte von polyzyklischen Estern, wobei keine weitere Geruchsbeschreibung der Verbindungen offenbart wird, noch wird ein Einsatz von diesen Verbindungen in Aroma- und/oder Riechstoffmischungen beschrieben.

In der Veröffentlichung TETRAH. ASYMMETRY, p 1385-1391 (2003**)** geht es um die Enantiomer-reine Darstellung von Tetrahydrofuran-2-carbonsäuren (THFC). Konkret werden die Verbindungen: Terahydrofuran-2-carbonsäuremethylester (S. 1386, Verbindung 3a), Terahydrofuran-2-carbonsäurebutylester (S. 1386, Verbindung 3b), Terahydrofuran-2-carbonsäureethylester (S. 1386, 3d) und Terahydrofuran-2-carbonsäureisopropylester (S. 1386, 3e), offenbart.

Die Schrift BULL. OF ACAD. OF S. OF THE USSR DIV. OF CHEM. SCIENCE, VOL. 31, p 1796-1798 (1982**)** befasst sich mit der Protonen-Akzeptor Eigenschaft der Carbonyleinheit in Tetrahydrofuransäurederivaten. Hierbei werden die Verbindungen Terahydrofuran-2-carbonsäureethylester (I), Terahydrofuran-2-carbonsäureisopropylester (II), Terahydrofuran-2-carbonsäurebutylester (III) Terahydrofuran-2-carbonsäureallyester (IV), und 1-(2'-tetrahydrofuryl)-1-propanone (X), offenbart.

Gegenstand der EP 1318148 A1 (SK CORP) ist die optisch reine Darstellung von (R)- oder (S)- Tetrahydrofuranderivaten, u.a. von 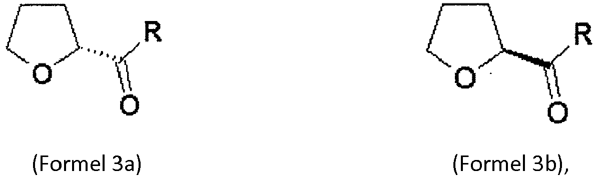 In Beispiel 1 wird (S)-2-Acetyl-tetrahydrofuran hergestellt.

Es besteht daher in der Parfümindustrie grundsätzlich ein ständiger Bedarf an neuen Riechstoffen, die sich zur Herstellung von Riechstoffkompositionen oder parfümierten Artikeln eignen. Insbesondere besteht ein Bedarf an Riechstoffen, die durch die oben erwähnten technischen Eigenschaften zu einem erhöhten Nutzen von Riechstoffkompositionen und Parfümölen führen.

Die Aufgabe der vorliegenden Erfindung hat daher darin bestanden, neue Riech- und Duftstoffe mit einem interessanten Geruchsprofil zu identifizieren. Die neuen Riech- und Duftstoffe sollen insbesondere eine frische und süßliche Note aufweisen, jedoch daneben auch noch weitere interessante geruchliche Noten und Aspekte aufzeigen, die ihnen mehr geruchlichen Charakter und Komplexität verleihen, insbesondere für den Einsatz in Aroma- und Duftstoffmischungen mit weiteren Riech- und Duftstoffen. Es war ebenfalls Aufgabe der vorliegenden Erfindung, Riech- und Dufstoffverbindungen zu identifizieren, die ein breites Geruchsprofil aufweisen, ohne negative Neben- oder Nachnoten, so dass diese breit einsetzbar sind. Insbesondere sollen die neuen Duftstoffe auch für den Einsatz im Bereich der Parfümerie geeinget sein. Vor allem war es Aufgabe der Erfindung, Riech- und Dufstoffverbindungen bereitzustellen, die möglichst toxisch unbedenklich sind, so dass sie geeignet sind für kosmetische Produkte und insbesondere für die Parfümerie, wo die Riech- und Dufstoffverbindungen direkten Kontakt mit der menschlichen Haut haben. Ebenfalls sollen die neuen Duftstoffe gute Stabilitäten und Kompatibilitäten mit (möglichst vielen) anderen Riechstoffen aufweisen.

### BESCHREIBUNG DER ERFINDUNG

Gegenstand der vorliegenden Erfindung sind Aroma- und/oder Riechstoffmischungen, umfassend
(i) ein oder mehrere Tetrahydrofuranderivat(e) der Formel (I) 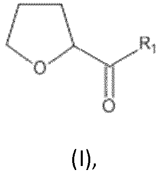
   worin R1 ein Wasserstoff, C1-C3- Alkyl oder einen Rest O-R2 darstellt,
   worin R2 eine verzweigte oder unverzweigte C1-C5-Alkylgruppe oder verzweigte oder unverzweigte C2-C6-Alkenylgruppe, eine substituierte oder unsubstituierte Cycloalkylgruppe, eine substituierte oder unsubstituierte (C₁-C₈) Alkyl (C₃-C₇) cycloalkylgruppe, eine substituierte oder unsubstituierte Cycloalkenylgruppe, eine substituierte oder unsubstituierte (C₂-C₈) Alkenyl (C₃-C₇) cycloalkylgruppe eine substituierte oder unsubstituierte Arylgruppe oder eine substituierte oder unsubstituierte (C1-C₈) Alkylarylgruppe, eine substituierte oder unsubstituierte (C₂-C₈) Alkenylarylgruppe darstellt, wobei alle Stereoisomere der Verbindung (I) eingeschlossen sind, und
(ii) einen oder mehrere weitere Riech- oder Aromastoffe,
   mit der Maßgabe, dass die Gesamtmenge an enthaltenen Tetrahydrofuranderivaten der Formel (I) bezogen auf die gesamte Aroma- oder Riechstoffmischung im Bereich von 0,00001 bis 40 Gew.-% liegt.

Im Sinne der vorliegenden Erfindung ist unter dem Begriff "verzweigte oder unverzweigte C1-C5-Alkylgruppe" ein Rest zu verstehen, welcher geradkettig oder verzweigt sein können und beispielsweise für eine Methyl-, Ethyl-, n-Propyl-, iso- Propyl-, n-Butyl, iso-Butyl, tert.-Butyl- oder n-Pentyl-, 2, 2-Dimethylpropyl-, 2-Methylbutyl- oder 3-Methylbutylgruppe stehen. Eine Methyl- oder Ethylgruppe ist bevorzugt.

Im Sinne der vorliegenden Erfindung ist unter dem Begriff "verzweigte oder unverzweigte C2-C6-Alkenylgruppe" ein Rest zu verstehen, welches eine Kohlenstoff-Kohlenstoff-Zweifachbindung (ethylenisches Strukturelement) enthält. Formal leitet sich ein Alkenylrest von einem Alken ab. Bevorzugte Alkylengruppe sind Ethylen, Propylen, Butylen (mit But-1-en, (Z)-But-2-en, (E)-But-2-en, 2-Methylprop-1-en), penten (mit Pent-1-en, cis-Pent-2-en, trans-Pent-2-en, 2-Methyl-but-1-en, 2-Methyl-but-2-en, 3-Methyl-but-1-en).

Im Sinne der vorliegenden Erfindung ist unter dem Begriff "Cycloalkylgruppe" eine gegebenenfalls durch ein oder mehrere Halogenatome substiuierte gesättigte zyklische Gruppe mit 3 bis 7 Ringkohlenstoffatomen, wie beispielsweise Cyclopropyl, Methylcyclopropyl, Cyclobutyl, Methylcyclobutyl, Cylopentyl, Methylcyclopentyl, Cyclohexyl, Methylcyclohexyl, Cycloheptyl, Methylcycloheptyl zu verstehen.

Im Sinne der vorliegenden Erfindung ist unter dem Begriff "Cycloalkenylgruppe", eine gegebenenfalls durch ein oder mehrere Halogenatome substiuierte ungesättigte zyklische Gruppe mit 3 bis 7 Ringkohlenstoffatomen zu verstehen.

Unter einer "(C₁-C₈) Alkyl (C₃-C₇) cycloalkylgruppe" ist ein Cycloalkyl-Gruppe zu verstehen, die über eine geradkettige oder verzweigte (C₁-C₈) -Alkyleinheit mit dem Sauerstoffatom, welches die Gruppe OR2 darstellt, verknüpft ist.

Unter einer "(C₂-C₈) Alkenyl (C₃-C₇) cycloalkylgruppe" ist ein Cycloalkyl-Gruppe zu verstehen, die über eine geradkettige oder verzweigte (C₂-C₈) -Alkenyleinheit mit dem Sauerstoffatom, welches die Gruppe OR2 darstellt, verknüpft ist.

Unter eine "Arylgruppe" sind aromatische oder teilaromatische carbocyclische Gruppen mit 6 bis 14 Kohlenstoffatomen zu verstehen, die einen Ring, wie z.B. Phenyl oder Phenylen oder mehrere kondensierte Ringe wie z.B. Napthyl oder Anthranyl aufweisen. Beispielhaft seien Phenyl, Naphthyl, Tetralinyl, Anthranyl, Indanyl, und Indenyl genannt. Die Arylgruppen können an jeder geeigneten Stelle, die zu einem stabilen Stereoisomeren führt, substituiert sein durch einen oder mehrere Reste aus der Gruppe Hydroxy oder Halogen .

Im Sinne der vorliegenden Erfindung ist unter dem Begriff "(C1-C₈) Alkylarylgruppe" eine Arylgruppe zu verstehen, wie sie oben bereits beschrieben ist, die über eine geradkettige oder verzweigte (C₁-C₈) -Alkyleinheit mit dem Sauerstoffatom, welches die Gruppe OR2 darstellt, verknüpft ist.

Im Sinne der vorliegenden Erfindung ist unter dem Begriff "(C₂-C₈) Alkenylarylgruppe" ist Arylgruppe zu verstehen, wie sie oben bereits beschrieben ist, die über eine geradkettige oder verzweigte (C₂-C₈) -Alkenyleinheit mit dem Sauerstoffatom, welches die Gruppe OR2 darstellt, verknüpft ist.

Unter dem Begriff "substiuiert oder unsubstituiert" ist zu verstehen, dass ein den jeweiligen Resten (beispielsweise am Arylrest oder am Cycloalkylring) gegebenenfalls weitere Reste sich befinden können.

Tetrahydrofuranderivate der Formel (I) sind bevorzugt ausgewählt aus der Gruppe bestehend aus:
Verbindung 1: Tetrahydrofuran-2-carbonsäuremethylester 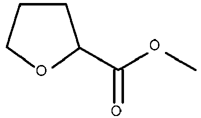
Verbindung 2: Tetrahydrofuran-2-carbonsäureethylester 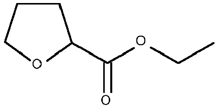
Verbindung 3: Tetrahydrofuran-2-carbonsäurepropylester 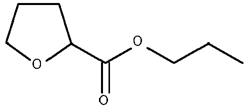
Verbindung 4: Tetrahydrofuran-2-carbonsäureallylester 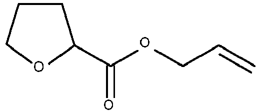
Verbindung 5: Tetrahydrofuran-2-carbonsäure-isopropylester 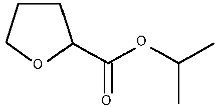
Verbindung 6: Tetrahydrofuran-2-carbonsäure-2-methylallylester 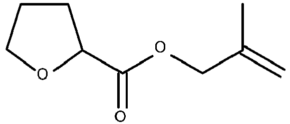
Verbindung 7: Tetrahydrofuran-2-carbonsäure-isobutylester 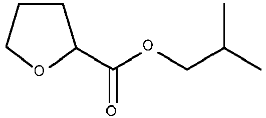
Verbindung 8: Tetrahydrofuran-2-carbonsäurebutylester 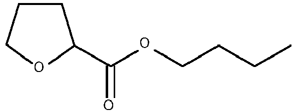
Verbindung 9: 1-(Tetrahydro-furan-2-yl)-ethanone

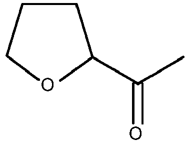

Die Verbindungen 6 und 7 stellen dabei ebenfalls Gegenstände der vorliegenden Erfindung dar.

### HERSTELLUNG VON TETRAHYDROFURANDERIVATE DER FORMEL (I)

Die Herstellung von Tetrahydrofurancarbonsäureestern der Formel (II) erfolgt gemäß US 2, 843, 607, in dem das Edukt (z.B. Furancarbonsäureestern oder Acetyllfuran, siehe Reaktion) unter H2-Atmosphäre im Autoklaven erhitzt und hydriert wird, wobei die Temperatur ca. bei 110°C gehalten wird. Die Reaktion dauert ca. 2 Stunden, wobei ein Ktaalysator, ein Raney-Nickel verwendet wird. Es ist von Vorteil bei der Reaktion einen weiteren Veresterungskatalysator, z.B. p-Toluolsulfonsäure, zu verwenden. Nach der Reaktion wird der Raney-nickel abgetrennt und das Reaktionsprodukt abdestilliert. In US 2,843,607 werden u.a. die Ethyl-, Propyl- und Heptylester der Tetrahydrofurancarbonsäure dargestellt. Es wird offenbart, dass die Tetrahydrofurancarbonsäurepropyl- und -heptylester als Lösungsmittel und auch in Parfüms eingesetzt werden können.

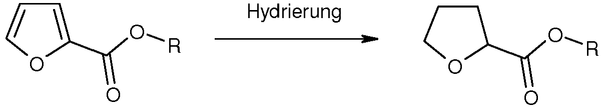

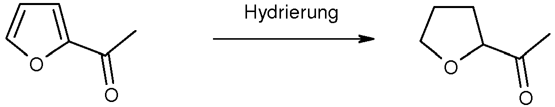

Eine detaillierte Geruchbeschreibung und Eignung als Riechstoff wird jedoch nicht angegeben. Ein Schwellenwert von dem Tetrahydrofurancarbonsäureethylester wird in einer Publikation angegeben (Takeoka, Teranishi and Buttery in Olfaction and Taste XI. Springer-Verlag, pp. 271-273), jedoch wird dort auch keine weitere Geruchsbeschreibung der Verbindung offenbart.

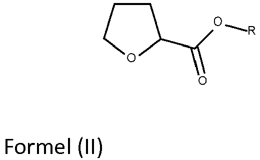

Die Verbindungen der allgemeinen Formel (I) können auch Umesterung von Tetrahydrofurancarbonsäuremethylester mit Alkoholen hergestellt werden:

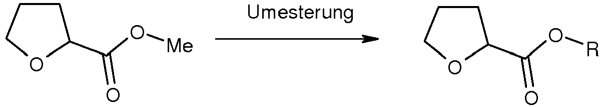

Überraschenderweise wurde gefunden, dass die Tetrahydrofuranderivate der Formel (I) in Aroma- und Riechstoffmischungen ein bestimmtes Geruchsprofil aufweisen, insbesondere die genannten Verbindungen 1 bis 9, welche bislang so nicht erkannt worden sind, und somit vor allem ihre Verwendung und Eignung als spezielle Riechstoffe, insbesondere für den Einsatz in definierten Parfümölen bisher nicht bekannt war.

Das sensorische Profil der einzelnen Verbindungen 1 bis 9 kann wie folgt beschrieben werden:

| **Verbindung** | **Formel** | **Geruchsnoten** |
|---|---|---|
| 1 | 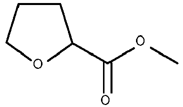 | frisch, kaffeeartig, |
| 2 | 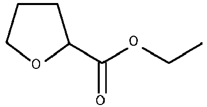 | frisch, süß, Rum, Whisky, Kaffee |
| 3 | 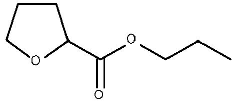 | frisch, süß, Rum, Kaffee |
| 4 | 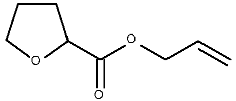 | frisch, süß, Rum, Kaffee |
| 5 | 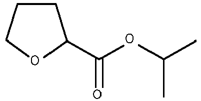 | frisch, grün, Rum |
| 6 | 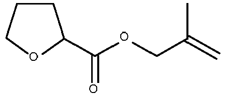 | frisch, süß, cremig, kaffeeartig |
| 7 | 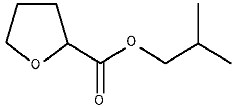 | frisch, süß, Rum, Kaffee |
| 8 | 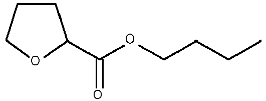 | cremig, süß, Rum, Kaffee |
| 9 | 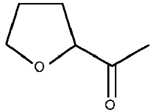 | grün, pilzig, cremig, kaffeeartig, |

In Mischungen mit anderen Riechstoffen vermögen Tetrahydrofuranderivate der Formel (I) und insbesondere die einzelnen Verbindungen 1 bis 9 oder Mischungen davon zudem bereits in geringen Dosierungen die Intensität einer Riechstoffmischung zu verstärken und das Gesamtbild der Riechstoffmischung geruchlich abzurunden sowie der Mischung mehr Ausstrahlung und Frische sowie Natürlichkeit zu verleihen. Zusammenfassend besitzen daher die Tetrahydrofuranderivate der Formel (I) und Mischungen davon eine überraschende geruchliche Qualität.

Die erfindungsgemäßen Aroma- und Riechstoffmischungen erlauben es, die oben genannten Vorteile zu verwirklichen und insbesondere Mischungen mit wertvollem frischem, süßlichem Geruch bzw. Charakter bereitzustellen.

Eine erfindungsgemäße Aroma- und Riechstoffmischung, vorzugsweise ein erfindungsgemäßes Parfümöl, umfasst Tetrahydrofuranderivate der Formel (I) oder Mischungen davon in einer Menge im Bereich von 0,00001 bis 40 Gew.-% liegt, bevorzugt von 0,001 bis 35 Gew.-% und besonders bevorzugt 0,2 bis 30 Gew.-%, jeweils bezogen auf das Gesamtgewicht der Aroma- und Riechstoffmischung, wobei die Menge, die Gesamtmenge aller Tetrahydrofuranderivate der Formel (I) ist.

Erfindungsgemäße Aroma- und Riechstoffmischungen, vorzugsweise Parfümöle, umfassen vorzugsweise weitere Riechstoffe, welche bevorzugt ausgewählt sind aus den genannten Stoffen in den nachfolgenden Veröffentlichungen: in "Riechstoffe", in Steffen Arctander, in "Perfume and Flavor Chemicals", Eigenverlag, Montclair, N. J. 1969; H. Surburg, J. Panten, in "Common Fragrance and Flavor Materials", 5th Edition, Wiley-VCH, Weinheim 2006.

Vorzugsweise werden in einer solchen Aroma- und/oder Riechstoffmischung eine Mischung von mehreren Tetrahydrofuranderivate der Formel (I) eingesetzt, bevorzugt zwei, drei, vier, fünf, sechs, sieben, acht oder neun Tetrahydrofuranderivate der Formel (I), vorzugsweise sind die Verbindungen ausgewählt aus den Verbindungen 1 bis 9, wobei die Isomeren Formen der Verbindungen ebenfalls mit umfasst sind.

Im Sinne der vorliegenden Erfindung wird unter eine Mischung von mehreren Tetrahydrofuranderivate der Formel (I), vorzugsweise mindestens ein, zwei, drei, vier, fünf, sechs, sieben, acht oder neun Verbindungen der Formel (I) zu verstehen, vorzugsweise sind die Verbindungen dann ausgewählt aus den Verbindungen 1 bis 9, wobei die Isomeren Formen der Verbindungen ebenfalls stets dadurch mitumfasst sind.

Im Sinne der vorliegenden Erfindung ist unter dem Begriff "Isomeren Formen der Tetrohydrofuranderivate der Formel (I)" die R- und S- Isomeren der Tetrahydrofuranderivate der Formel (I) zu verstehen. Die Verbindungen 1 bis 9 weisen alle jeweils ein Stereozentrum auf:
Verbindung 1: Tetrahydrofuran-2-carbonsäuremethylester 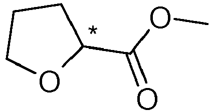
Verbindung 2: Tetrahydrofuran-2-carbonsäureethylester 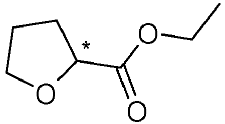
Verbindung 3: Tetrahydrofuran-2-carbonsäurepropylester 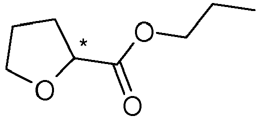
Verbindung 4: Tetrahydrofuran-2-carbonsäureallylester 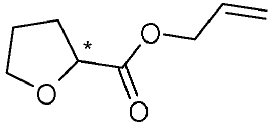
Verbindung 5: Tetrahydrofuran-2-carbonsäure-isopropylester 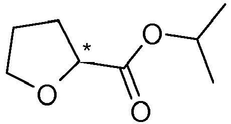
Verbindung 6: Tetrahydrofuran-2-carbonsäure-2-methylallylester 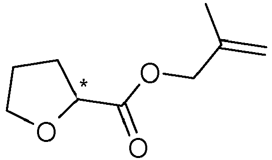
Verbindung 7: Tetrahydrofuran-2-carbonsäure-isobutylester 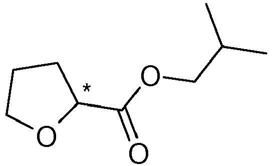
Verbindung 8: Tetrahydrofuran-2-carbonsäurebutylester 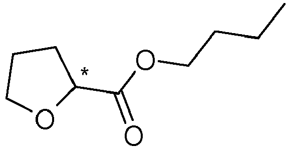
Verbindung 9: 1-(Tetrahydro-furan-2-yl)-ethanone

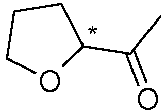

Entsprechend ist unter den Isomeren Formen der Verbindungen 1 bis 9, vorzugsweise die jeweiligen R- und S- Isomeren wie folgt zu verstehen:
Bei Verbindung 1:
   *R*- Tetrahydrofuran-2-carbonsäuremethylester (Verbindung 1a),
   *S*- Tetrahydrofuran-2-carbonsäuremethylester (Verbindung 1b),
Bei Verbindung 2:
   *R*- Tetrahydrofuran-2-carbonsäureethylester (Verbindung 2a),
   *S*- Tetrahydrofuran-2-carbonsäureethylester (Verbindung 2b),
Bei Verbindung 3:
   *R*- Tetrahydrofuran-2-carbonsäurepropylester (Verbindung 3a),
   *S*- Tetrahydrofuran-2-carbonsäurepropylester (Verbindung 3b),
Bei Verbindung 4:
   R- Tetrahydrofuran-2-carbonsäureallylester (Verbindung 4a),
   *S*- Tetrahydrofuran-2-carbonsäureallylester (Verbindung 4b),
Bei Verbindung 5:
   *R*- Tetrahydrofuran-2-carbonsäure-isopropylester (Verbindung 5a),
   *S*- Tetrahydrofuran-2-carbonsäure-isopropylester(Verbindung 5b),
Bei Verbindung 6:
   *R*- Tetrahydrofuran-2-carbonsäure-2-methylallylester (Verbindung 6a),
   *S*- Tetrahydrofuran-2-carbonsäure-2-methylallylester (Verbindung 6b),
Bei Verbindung 7:
   *R*- Tetrahydrofuran-2-carbonsäure-isobutylester (Verbindung 7a),
   *S*- Tetrahydrofuran-2-carbonsäure-isobutylester (Verbindung 7b),
Bei Verbindung 8:
   *R*- Tetrahydrofuran-2-carbonsäurebutylester (Verbindung 8a),
   *S*- Tetrahydrofuran-2-carbonsäurebutylester (Verbindung 8b),
Bei Verbindung 9:
   *R*- 1-(Tetrahydro-furan-2-yl)-ethanone (Verbindung 9a),
   *S*- 1-(Tetrahydro-furan-2-yl)-ethanone (Verbindung 9b).

Bei den kosmetishen Produkten und Haushaltsprodukten, die ebenfalls Gegenstand der vorliegenden Erfindung sind, handelt es sich bevorzugt um Wasch- und Reinigungsmittel, Hygiene- oder Pflegeprodukte handelt. Bevorzugt handelt es sich um Produkte für den Bereich der Körper- und/ oder Haarpflege, Shampoos, Weichspüler oder Waschpulver.

Ein weiterer wichtiger Aspekt der vorliegenden Erfindung ist die Verwendung von Tetrahydrofuranderivate der Formel (I) oder Mischungen davon
(i) zum Vermitteln, Verstärken, Verbessern und/oder Modifizieren eines Geruchseindrucks, und/oder
(ii) zum Erhöhen der Substantivität oder Diffusität eines Riechstoffs oder einer Aroma- und/oder Riechstoffmischung.

Insbesondere ist ein Gegenstand der vorliegenden Erfindung die Verwendung von Tetrahydrofuranderivate der Formel (I) oder Mischungen davon,
(i) zum Vermitteln, Verstärken, Verbessern und/oder Modifizieren eines Geruchseindrucks in Richtung von Reinheit, Frische und/oder Natürlichkeit, und/oder
(ii) zum geruchlichen Abrunden einer Aroma- und / oder Riechstoffmischung oder eines kosmetischen oder Haushaltsproduktes.

Ebenfalls ist ein Gegenstand der vorliegenden Erfindung die Verwendung von Tetrahydrofuranderivat der Formel (I) oder Mischungen davon, zum Vermitteln, Verstärken, Verbessern und/oder Modifizieren eines Geruchseindrucks in Richtung Volumen, Komplexität, Eleganz und / oder Natürlichkeit.

Ferner ist ein Gegenstand der vorliegenden Erfindung die Verwendung von Tetrahydrofuranderivaten der Formel (I) oder Mischungen davon, in Aroma- und/oder Riechstoffmischungen zum Vermitteln eines pflegenderen, harmonischeren, hochwertigeren und/ oder natürlicheren Geruchseindrucks in der Mischung im Vergleich zu einer Mischung, die keine Tetrahydrofuranderivate der Formel (I) oder Mischungen davon enthalten.

Ein weitere Aspekt der vorliegenden Erfindung ist die Verwendung der erfindungsgemäßen von Aroma- und/oder Riechstoffmischungen in kosmetischen Produkten oder Haushaltsprodukten
(i) zur Vermittlung, Verstärkung, Verbesserung und/oder Modifizierung eines Geruchseindrucks, und/oder
(ii) zur Erhöhung der Substantivität oder Diffusität eines Riechstoffs oder der Aroma- und/oder Riechstoffmischung und / oder
(iii) zur Vermittlung, Verstärkung, Verbesserung und/oder Modifizierung eines Geruchseindrucks in Richtung von Reinheit, Frische und/oder Natürlichkeit, und / oder
(iv) zur geruchlichen Abrundung der Aroma- und/oder Riechstoffmischung oder eines kosmetischen oder Haushaltsproduktes.

In den erfindungsgemäßen Aroma- und Riechstoffmischungen und (kosmetischen und Haushalts-) Produkten werden die Tetrahydrofuranderivate der Formel (I) vorzugsweise mit weiteren Riech- und Duftstoffe eingesetzt. Geeignete vorzugsweise kombinierbare Riechstoffe sind hierbei ausgewählt aus:
- Extrakten aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-Citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl, Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-Cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-Tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon bzw. daraus isolierte Inhaltsstoffe;
- einzelnen Riechstoffe aus der Gruppe der Kohlenwasserstoffe, wie z. B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (*E,Z*)-1,3,5-Undecatrien;
- der Gruppe der aliphatischen Alkohole, wie z. B. Hexanol; Octanol; 3-Octanol; 2,6-Dimethylheptanol; 2-Methylheptanol, 2-Methyloctanol; (*E*)-2-Hexenol; (*E*)- und (*Z*)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (*E,Z*)-2,6-Nonadienol; 3,7-Dimethyl-7-methoxyoctan-2-ol; 9-Decenol; 10-Undecenol; 4-Methyl-3-decen-5-ol;
- der Gruppe der aliphatischen Aldehyde und deren Acetale wie z. B. Hexanal; Heptanal; Octanal; Nonanal; Decanal; Undecanal; Dodecanal; Tridecanal; 2-Methyloctanal; 2-Methylnonanal; (*E*)-2-Hexenal; (*Z*)-4-Heptenal; 2,6-Dimethyl-5-heptenal; 10-Undecenal; (*E*)-4-Decenal; 2-Dodecenal; 2,6,10-Trimethyl-5,9-undecadienal; Heptanal-diethylacetal; 1,1-Dimethoxy-2,2,5-trimethyl-4-hexen; Citronellyloxyacetaldehyd;
- der Gruppe der aliphatischen Ketone und deren Oxime wie z. B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on;
- der Gruppe der aliphatischen schwefelhaltigen Verbindungen wie z. B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
- der Gruppe der aliphatischen Nitrile wie z. B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
- der Gruppe der aliphatischen Carbonsäuren und deren Ester wie z. B. (*E*)- und (*Z*)-3-Hexenylformiat; Ethylacetoacetat; Isoamylacetat; Hexylacetat; 3,5,5-Trimethylhexylacetat; 3-Methyl-2-butenylacetat; (*E*)-2-Hexenylacetat; (*E*)- und (*Z*)-3-Hexenylacetat; Octylacetat; 3-Octylacetat; 1-Octen-3-ylacetat; Ethylbutyrat; Butylbutyrat; Isoamylbutyrat; Hexylbutyrat; (*E*)- und (*Z*)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(*E,Z*)-2,4-decadienoat; Methyl-2-octinat; Methyl-2-noninat; Allyl-2-isoamyloxyacetat; Methyl-3,7-dimethyl-2,6-octadienoat;
- der Gruppe der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
- der Gruppe der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
- der Gruppe der cyclischen Terpenalkohole wie z. B. Menthol; Isopulegol; α-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
- der Gruppe der cyclischen Terpenaldehyde und -ketone wie z. B. Menthon; Isomenthon; 8-Mercaptomenthan-3-on; Carvon; Campher; Fenchon; α-Ionon; β-Ionon; α-n-Methylionon; β-n-Methylionon; α-Isomethylionon; β-Isomethylionon; α-Iron; α-Damascon; β-Damascon; β-Damascenon; γ-Damascon; δ-Damascon; 1-(2,4,4-Trimethyl-2-cyclohexen-1-yl)-2-buten-1-on; 1,3,4,6,7,8a-Hexahydro-1,1,5,5-tetramethyl-2*H*-2,4a-methanonaphthalen-8(5*H*)-on; Nootkaton; Dihydronootkaton; α-Sinensal; β-Sinensal; acetyliertes Cedernholzöl (Methylcedrylketon);
- der Gruppe der cyclischen Alkohole wie z. B. 4-*tert*-Butylcyclohexanol; 3,3,5-Trimethylcyclohexanol; 3-Isocamphylcyclohexanol; 2,6,9-Trimethyl-(*Z2,Z5,E9*)-cyclododecatrien-1-ol; 2-Isobutyl-4-methyltetrahydro-2*H*-pyran-4-ol;
- der Gruppe der cycloaliphatischen Alkohole wie z. B. α,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
- der Gruppe der cyclischen und cycloaliphatischen Ether wie z. B. Cineol; Cedrylmethylether; Cyclododecylmethylether; (Ethoxymethoxy)cyclododecan; α-Cedrenepoxid; 3a,6,6,9a-Tetramethyldodecahydronaphtho[2,1-b]furan; 3a-Ethyl-6,6,9a-trimethyl-dodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
- der Gruppe der cyclischen Ketone wie z. B. 4-tert-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-*cis*-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-*tert*-Pentylcyclohexanon; 5-Cyclohexadecen-1-on; 6,7-Dihydro-1,1,2,3,3-pentamethyl-4(5*H*)-indanon; 9-Cycloheptadecen-1-on; Cyclopentadecanon; Cyclohexadecanon;
- der Gruppe der cycloaliphatischen Aldehyde wie z. B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
- der Gruppe der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; *tert*-Butyl-(2,4-dimethyl-3-cyclohexen-1-yl)keton;
- der Gruppe der Ester cyclischer Alkohole wie z. B. 2-*tert*-Butylcyclohexylacetat; 4*-tert-*Butylcyclohexylacetat; 2-*tert*-Pentylcyclohexylacetat; 4-*tert*-Pentylcyclohexylacetat; Decahydro-2-naphthylacetat; 3-Pentyltetrahydro-2*H*-pyran-4-ylacetat; Decahydro-2,5,5,8a-tetramethyl-2-naphthylacetat; 4,7-Methano-3a,4,5,6,7,7a-hexahydro-5- bzw. -6-indenylacetat; 4,7-Methano-3a,4,5,6,7,7a-hexahydro-5- bzw. -6-indenylpropionat; 4,7-Methano-3a,4,5,6,7,7a-hexahydro-5- bzw. -6-indenylisobutyrat; 4,7-Methanooctahydro-5- bzw. -6-indenylacetat;
- der Gruppe der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
- der Gruppe der aromatischen Kohlenwasserstoffe wie z. B. Styrol und Diphenylmethan;
- der Gruppe der araliphatischen Alkohole wie z. B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
- der Gruppe der Ester von araliphatischen Alkoholen und aliphatischen Carbonsäuren wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; α-Trichlormethylbenzylacetat; α,α-Dimethylphenylethylacetat; α,α-Dimethylphenylethylbutyrat; Cinnamylacetat; 2-Phenoxyethylisobutyrat; 4-Methoxybenzylacetat;
- der Gruppe der araliphatischen Ether wie z. B. 2-Phenylethylmethylether; 2-Phenylethylisoamylether; 2-Phenylethyl-1-ethoxyethylether; Phenylacetaldehyddimethylacetal; Phenylacetaldehyd-diethylacetal; Hydratropaaldehyd-dimethylacetal; Phenylacetaldehyd-glycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
- der Gruppe der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-*tert*-butylphenyl)propanal; 3*-*(4*-tert-*Butylphenyl)propanal; Zimtaldehyd; α-Butylzimtaldehyd; α-Amylzimtaldehyd; α-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
- der Gruppe der aromatischen und araliphatischen Ketone wie z. B. Acetophenon; 4-Methylacetophenon; 4-Methoxyacetophenon; 4-*tert*-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-*tert*-Butyl-1,1-dimethyl-4-indanylmethylketon; 1-[2,3-dihydro-1,1,2,6-tetramethyl-3-(1-methylethyl)-1*H*-5-indenyl]ethanon; 5',6',7',8'-Tetrahydro-3',5',5',6',8',8'-hexamethyl-2-acetonaphthon;
- der Gruppe der aromatischen und araliphatischen Carbonsäuren und deren Ester wie z. B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; *cis*-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
- der Gruppe der stickstoffhaltigen aromatischen Verbindungen wie z. B. 2,4,6-Trinitro-1,3-dimethyl-5-*tert*-butylbenzol; 3,5-Dinitro-2,6-dimethyl-4-*tert*-butylacetophenon; Zimtsäurenitril; 5-Phenyl-3-methyl-2-pentensäurenitril; 5-Phenyl-3-methylpentansäurenitril; Methylanthranilat; Methy-*N*-methylanthranilat; Schiff'sche Basen von Methylanthranilat mit 7-Hydroxy-3,7-dimethyloctanal, 2-Methyl-3-(4-*tert*-butylphenyl)propanal oder 2,4-Dimethyl-3-cyclohexencarbaldehyd; 6-Isopropylchinolin; 6-Isobutylchinolin; 6*-sec-*Butylchinolin; Indol; Skatol; 2-Methoxy-3-isopropylpyrazin; 2-Isobutyl-3-methoxypyrazin; 4-(4,8-Dimethyl-3,7-nonadienyl)-pyridin;
- der Gruppe der Phenole, Phenylether und Phenylester wie z. B. Estragol; Anethol; Eugenol; Eugenylmethylether; Isoeugenol; Isoeugenylmethylether; Thymol; Carvacrol; Diphenylether; β-Naphthylmethylether; β-Naphthylethylether; β-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
- der Gruppe der heterocyclischen Verbindungen wie z. B. 2,5-Dimethyl-4-hydroxy-2*H-*furan-3-on; 2-Ethyl-4-hydroxy-5-methyl-2*H*-furan-3-on; 3-Hydroxy-2-methyl-4*H*-pyran-4-on; 2-Ethyl-3-hydroxy-4*H*-pyran-4-on;
- der Gruppe der Lactone wie z. B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; *cis-* und *trans*-11-Pentadecen-1,15-olid; *cis-* und *trans*-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Blumige Riech- oder Aromastoffe, mit denen die Tetrahydrofuranderivate der Formel (I), vorteilhafterweise kombiniert werden können, werden vorzugsweise gewählt aus der Gruppe bestehend aus: Hydroxycitronellal, Methoxycitronellal, Cyclamenaldehyd [2-Methyl-3-(4-isopropylphenyl)propanal], 1-(4-Isopropyl-cyclohexyl)ethanol (Mugetanol(R)), 4-tert.-Butyl-a-methyldihydrozimtaldehyd (Lilial(R)), cis-Hexahydrocuminylalkohol (Mayol(R)), 3-[4-(1,1-Dimethylethyl)phenyl]propanal (Bourgeonal(R)), 2,2-Dimethyl-3-(3-methylphenyl)propanol (Majantol(R)), 3-Methyl-3-(3-methylbenzyl)-butan-2-ol, 2-Isobutyl-4-methyltetrahydro-2H-pyran-4-ol (Florosa(R)), 2-Methyl-3-(3,4-methylendioxyphenyl)propanal (Heliofolal(R)), 4-(4-Hydroxy-4-methylpentyl)-3-cyclohexencarbaldehyd (Lyral(R)), 4-(Octahydro-4,7-methano-5H-inden-5-yliden-butanal (Dupical(R)), Vernaldehyd, 4-(4-Methyl-3-penten-1-yl)-3-cyclohexencarbaldehyd (Vertomugal(R)), Octahydro-5-(4-methoxybutyliden)-4,7-methano-1H-inden (Mugoflor(R)), 2,6-Dimethyl-2-heptanol (Freesiol(R)), 1-Ethyl-1-methyl-3-phenylpropanol (Phemec(R)), 2,2-Dimethyl-3-phenyl-1-propanol (Muguet alcohol), Profarnesol, Dihydrofarnesol, Farnesol, Nerolidol, Hydroxycitronellaldimethylacetal, Hexylbenzoat, Geraniol, Nerol, Linalool, Tetrahydrogeraniol, Tetrahydrolinalool, Ethyllinalool, Geranyltiglinat, Phenethylalkohol (2-Phenylethylalkohol), Citronellol, Rosenoxid, 2-Methyl-5-phenylpentanol (Rosaphen), 3-Methyl-5-phenylpentanol (Phenoxanol), Methyldihydrojasmonat (Hedion(R), Hedione(R) high cis), 2-Heptylcyclopentanon (Projasmon P), cis-Jasmon, Dihydrojasmon, Zimtalkohol (3-Phenyl-2-propen-1-ol), Dihydrozimtalkohol (3-Phenylpropanol), 2-Methyl-4-phenyl-1,3-dioxolan (Jacinthaflor(R)), Dihydromyrcenol (2,6-Dimethyl-7-octen-2-ol).

Fruchtige Riech- oder Aromastoffe, mit denen die Tetrahydrofuranderivate der Formel (I), vorteilhafterweise kombiniert werden können, werden vorzugsweise gewählt aus der Gruppe bestehend aus: 2-Methyl-buttersaeureethylester, 4-(p-Hydroxyphenyl)-2-butanon, Ethyl-3-methyl-3-phenylglycidat, Buttersäureisoamylester, Essigsäureisoamylester, Essigsäure-n-butylester, Buttersäureethylester, 3-Methyl-buttersaeureethylester, n-Hexansäureethylester, n-Hexansäureallylester, Ethyl-2-trans-4-cis-decadienoat, 1,1-Dimethoxy-2,2,5-trimethyl-4-hexan, 2,6-Dimethyl-5-hepten-1-al, gamma-Undecalacton, gamma-Nonalacton, Hexanal, 3Z-Hexenal, n-Decanal, n-Dodecanal, Citral, Limonen, Vanillin, Ethylvanillin, Maltol, Ethylmaltol und deren Mischungen.

Wie bereits erwähnt, eignet sich die erfindungsgemäßen Verbindungen wegen ihrer olfaktorischen Eigenschaften besonders gut für den Einsatz in Riechstoffmischungen und Parfümölen. Die Verbindungen können dabei alleine oder in erfindungsgemäßen Mischungen in entsprechenden Riechstoffmischungen mit einem weiteren Einzelriechstoff oder aber einer Vielzahl weiterer Riechstoffe zusammen eingesetzt werden. Besonders vorteilhaft lässt sich die erfindungsgemäßen Verbindungen mit anderen Riechstoffen, vorzugsweise ausgewählt aus den bereits oben bzw. weiter unten genannten Riechstoffen, in unterschiedlichen Mengenverhältnissen zu neuartigen Riechstoffmischungen bzw. Parfümen kombinieren.

Erfindungsgemäß werden daher Aromastoffmischungen und Riechstoffmischungen (Parfümöle) bereitgestellt, die neben weiteren Duft- und Riechstoffe ein oder mehrere Tetrahydofuranderivate der Formel (I) umfassen. Dabei sind insbesondere einzelne oder Mischungen der Tetrahydrofuranderivate *R*- Tetrahydrofuran-2-carbonsäuremethylester (Verbindung 1a), *S*- Tetrahydrofuran-2-carbonsäuremethylester (Verbindung 1b), *R*- Tetrahydrofuran-2-carbonsäureethylester (Verbindung 2a), *S*- Tetrahydrofuran-2-carbonsäureethylester (Verbindung 2b), *R*- Tetrahydrofuran-2-carbonsäurepropylester (Verbindung 3a), *S*- Tetrahydrofuran-2-carbonsäurepropylester (Verbindung 3b), *R-* Tetrahydrofuran-2-carbonsäureallylester (Verbindung 4a), *S*- Tetrahydrofuran-2-carbonsäureallylester (Verbindung 4b), *R*- Tetrahydrofuran-2-carbonsäure-isopropylester (Verbindung 5a), *S*- Tetrahydrofuran-2-carbonsäure-isopropylester(Verbindung 5b), *R*- Tetrahydrofuran-2-carbonsäure-2-methylallylester (Verbindung 6a), *S*- Tetrahydrofuran-2-carbonsäure-2-methylallylester (Verbindung 6b), *R-* Tetrahydrofuran-2-carbonsäure-isobutylester (Verbindung 7a), *S*- Tetrahydrofuran-2-carbonsäure-isobutylester (Verbindung 7b), *R-* Tetrahydrofuran-2-carbonsäurebutylester (Verbindung 8a), *S*- Tetrahydrofuran-2-carbonsäurebutylester (Verbindung 8b), *R-* 1-(Tetrahydro-furan-2-yl)-ethanone (Verbindung 9a), *S*- 1-(Tetrahydro-furan-2-yl)-ethanone (Verbindung 9b) bevorzugt. Besonders bevorzugt sind einzelne oder Mischungen der Tetrahydrofuranderivate *R-* Tetrahydrofuran-2-carbonsäuremethylester (Verbindung 1a), *S*- Tetrahydrofuran-2-carbonsäuremethylester (Verbindung 1b), *R*- Tetrahydrofuran-2-carbonsäureethylester (Verbindung 2a), *S*- Tetrahydrofuran-2-carbonsäureethylester (Verbindung 2b), *R-* Tetrahydrofuran-2-carbonsäurepropylester (Verbindung 3a), *S*- Tetrahydrofuran-2-carbonsäurepropylester (Verbindung 3b), *R-* Tetrahydrofuran-2-carbonsäureallylester (Verbindung 4a), *S*- Tetrahydrofuran-2-carbonsäureallylester (Verbindung 4b). Ganz besonder bevorzugt sind einzelne oder Mischungen der Tetrahydrofuranderivate *R*- Tetrahydrofuran-2-carbonsäuremethylester (Verbindung 1a), *S*- Tetrahydrofuran-2-carbonsäuremethylester (Verbindung 1b), *R-* Tetrahydrofuran-2-carbonsäureethylester (Verbindung 2a), *S*- Tetrahydrofuran-2-carbonsäureethylester (Verbindung 2b).

Tetrahydrofuranderivate der Formel (I) entfalten ihre vorteilhaften Wirkungen zudem vorzugsweise in Produkten enthaltend diese Substanzen. Erfindungsgemäß werden deshalb auch aromatisierte oder parfümierte Produkte bereitgestellt, enthaltend eine erfindungsgemäße Aroma- oder Riechstoffmischung (Parfümöl) mit einem oder mehreren Tetrahydrofuranderivaten der Formel (I). Zweckmäßigerweise liegt dabei zumindest eines der Tetrahydrofuranderivaten der Formel (I) in einer sensorisch ausreichenden Menge zum Hervorbringen des erfindungsgemäß vorteilhaften Geruchseindrucks der jeweiligen Substanz vor. Insbesondere ist es zweckmäßig, wenn das eine oder mehrere Tetrahydrofuranderivate der Formel (I) in einer zum Hervorbringen eines wässrigen Geruchs ausreichenden Menge vorliegt.

Eine sensorisch ausreichende Menge zum Hervorbringen eines Geruchseindrucks kann der Fachmann leicht in Ansehung der weiteren Bestandteile des Produkts durch ein Panel aus acht Prüfern ohne Anosmie für das jeweilige Tetrahydrofuranderivat der Formel (I) bestimmen, in die eine entsprechende Probe des Produkts mit unterschiedlichen Konzentrationen an Tetrahydrofuranderivaten der Formel (I)dreimal von jedem Panelmitglied bei Raumtemperatur auf das Vorliegen der jeweils gewünschten sensorischen Eigenschaft hin untersucht wird. Die minimale Konzentration ist dann erreicht, wenn zumindest vier Mitglieder des Panels das Vorliegen der gewünschten sensorischen Eigenschaft attestieren. Derartige Panelversuche sind Parfümeuren und Floristen geläufig und werden routinemäßig standardisiert eingesetzt.

Die Tetrahydrofuranderivate der Formel (I), erfindungsgemäße Mischungen davon bzw. erfindungsgemäße Aroma- und Riechstoffmischungen werden vorzugsweise zur Herstellung parfümierter Produkte (parfümierte Artikel) eingesetzt. Die sensorischen Eigenschaften ebenso wie die stofflichen Eigenschaften (wie Löslichkeit in gängigen Lösungsmitteln und Kompatibilität mit gängigen weiteren Bestandteilen derartiger Produkte) sowie die toxikologische Unbedenklichkeit der erfindungsgemäßen Verbindungen unterstreichen ihre besondere Eignung für die genannten Einsatzzwecke.

Parfümierte Produkte im Rahmen der vorliegenden Erfindung, sind unterschiedliche Produkte beispielsweise aus den Bereichen Wasch- und Reinigungsmittel, kosmetische Produkte, Parfümartikel sowie kosmetische Reinigungsmittel, die vorzugsweise ausgewählt sind aus der Gruppe bestehend aus:

Parfüm-Extraits, Eau de Parfums, Eau de Toilettes, Rasierwässer, Eau de Colognes, Pre-shave-Produkte, Splash-Colognes, parfümierte Erfrischungstücher, Parfüms für saure, alkalische und neutrale Reinigungsmittel, Waschmittel, Waschtabletten, Desinfektionsmitteln, sowie von Luftverbesserern, Aerosolsprays, Wachse und Polituren, sowie Körperpflegemitteln, Badeölen, kosmetischen Emulsionen, wie z.B. Hautcremes- und -lotionen, Sonnenschutzcremes- und -lotionen, After-sun-cremes und -lotionen, Handcremes und - lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigende Haarlotionen, Haarspülungen, Haarfärbemitteln, Haarverformungsmitteln und Haarglättungsmitteln, Haarwässern, Haarcremes und -lotionen, Deodorantien und Antiperspirantiens, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Erfindungsgemäße Aroma- und Riechstoffmischungen enthaltend die erfindungsgemäßen Verbindungen oder eine wie oben definierte erfindungsgemäße Mischung können allgemein (z.B. in konzentrierter Form, in Lösungen oder in unten beschriebener modifizierter Form) verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-ÖI- und vom Wasser-in-ÖI-in-Wasser-Typ wie z.B. Hautcremes- und -lotionen, Gesichtscremes und -lotionen, Sonnenschutzcremes und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Erfindungsgemäße Aroma- und Riechstoffmischungen enthaltend die erfindungsgemäßen Verbindungen oder eine wie oben definierte erfindungsgemäße Mischung können in flüssiger Form, unverdünnt oder mit einem Lösungsmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw. Für die genannten Lösungsmittel gilt, dass diese im Rahmen des vorliegenden Textes im Falle des Vorhandenseins eigener olfaktorischer Eigenschaften ausschließlich dem Bestandteil "Lösungsmittel" und nicht den "Riechstoffen" zuzuordnen sind.

Die in den erfindungsgemäßen parfümierten Produkten enthaltene erfindungsgemäßen Verbindungen, eine wie oben definierte erfindungsgemäße Mischung oder eine wie oben definierte erfindungsgemäße Aroma- und Riechstoffmischung können dabei in einer bevorzugten Ausführungsform an einem Trägerstoff absorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die in den erfindungsgemäßen parfümierten Produkten enthaltene erfindungsgemäße Verbindungen, eine wie oben definierte erfindungsgemäße Mischung oder eine wie oben definierte erfindungsgemäße Riechstoffmischung können auch mikroverkapselt, sprühgetrocknet, als Einschluss-Komplexe oder als Extrusions-Produkte vorliegen und in dieser Form dem zu parfümierenden Produkt, bzw. Artikel, hinzugefügt werden. Gegebenenfalls können die Eigenschaften von derart modifizierten Aroma- und Riechstoffmischungen durch so genanntes "Coaten" mit geeigneten Materialien im Hinblick auf eine gezieltere Duftstofffreisetzung weiter optimiert werden, wozu vorzugsweise wachsartige Kunststoffe wie z.B. Polyvinylalkohol verwendet werden.

Die Mikroverkapselung der Riechstoffmischungen kann beispielsweise durch das sogenannte Koazervationsverfahren mit Hilfe von Kapselmaterialien, z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riechstoffmischung und Cyclodextrinen oder Harnstoffderivaten in ein geeignetes Lösungsmittel, z.B. Wasser, hergestellt werden. Extrusions-Produkte können durch Verschmelzen der Riechstoffmischung mit einem geeigneten wachsartigen Stoff und durch Extrusion mit nachfolgender Erstarrung, ggf. in einem geeigneten Lösungsmittel, z.B. Isopropanol, erfolgen.

Die erfindungsgemäßen Aroma- und Riechstoffmischungen können demnach, wie bereits erwähnt, in konzentrierter Form, in Lösungen oder in oben beschriebener modifizierter Form für die Herstellung der entsprechenden erfindungsgemäßen parfümierten Artikel verwendet werden

In Riechstoff- und Aromastoffmischungen, insbesondere in Parfümöl-Kompositionen, liegt die eingesetzte Gesamtmenge an Tetrahydrofuranderivaten der Formel (I) vorzugsweise im Bereich von 0,000001 bis 40 Gew.- %, bevorzugt von 0,001 bis 35 Gew.-% und besonders bevorzugt 0,2 bis 30 Gew.-%, jeweils bezogen auf die gesamte Riechstoff- oder Aromastoffmischung.

In geringen Dosierungen kann gemäß einem weiteren Aspekt der Erfindung die erfindungsgemäß in den Aroma- und/oer Riechstoffmischungen einzusetzenden Tetrahydrofuranderivate der Formel (I), insbesondere in den nachfolgend genannten geringen Konzentrationen hervorragend zum Modifizieren und/oder Verstärken eines Geruchs- oder Geschmacks geeignet sein, d.h. dass sie insbesondere als sogenannte Booster oder Enhancer fungieren Können.

Sofern die Tetrahydrofuranderivate der Formel (I) hauptsächlich eingesetzt werden um einer Riechstoff- und Aromastoffmischung, insbesondere einer Parfümöl-Komposition, mehr Intensität, Frische, (Aus)Strahlung und/oder Abrundung zu verleihen und/oder bestimmte Noten (anderer in der Riechstoff- und Aromastoffmischung enthaltenen Riech- oder Aromastoffe) zu verstärken, insbesondere Noten der Richtungen frisch und süß ist der Gesamtanteil Tetrahydrofuranderivate der Formel (I) vorzugsweise recht niedrig und liegt vorzugsweise im Bereich von 0,00001 bis 5 Gew.- %, bevorzugt im Bereich von 0,0001 bis 3 Gew.- % und besonders bevorzugt im Bereich von 0,2 bis 2 Gew.- %, jeweils bezogen auf die Gesamtmenge der Riechstoff- oder Aromastoffmischung.

Für einen Geruchseindruck, welche einen Riechstoff- und Aromastoffmischung, insbesondere einer Parfümöl-Komposition, mehr in Richtung Kaffee und Rum verleiht, werden die erfindungsgemäßen Tetrahydrofuranderivate der Formel (I) vorzugsweise im höheren Bereichen eingesetzt, wie beispielsweise von 6 bis 30 Gew.- %, bevorzugt im Bereich von 6 bis 20 Gew.- % und besonders bevorzugt im Bereich von 6 bis 15 Gew.- %, jeweils bezogen auf die Gesamtmenge der Riechstoff- oder Aromastoffmischung.

Wie oben bereits ausgeführt, ist für tensidhaltige parfümierte Produkte die Substantivität eines Riechstoffs bzw. einer Riechstoffmischung gegenüber den bzw. deren Retention am Substrat, insbesondere Haaren oder textilen Fasern, eine weitere wichtige anwendungstechnische Anforderung.

Durch Zusatz der Tetrahydrofuranverbindungen bzw. einer entsprechenden Mischung der Verbindungen zu einer vorgegebenen Riechstoffmischung nur geringer Substantivität und/oder Retention werden diese Eigenschaften in besonders vorteilhafter Weise verbessert. So lässt sich beispielsweise eine zwar fruchtig, blumig, und/oder würzig duftende, aber aufgrund nur mangelhafter Substantivität der enthaltenen Duftstoffe nicht zum Weitergeben eines fruchtigen, blumigen und/oder würzigen Geruchs an Wäsche (textile Fasern) oder Haare geeignete wässrige Waschlösung (bzw. ein entsprechendes Waschmittel oder Shampoo oder dergleichen) durch Zusatz der erfindungsgemäßen Verbindungen bzw. einer erfindungsgemäßen Mischung in eine Lösung überführen, die einen fruchtigen, blumigen und/oder würzigen Geruch hervorragend weitergibt - und an den behandelten Substraten (Haare bzw. textile Fasern) haftet der fruchtige, blumige und/oder würzige Geruch lange an. Die erfindungsgemäßen Verbindungen bzw. eine erfindungsgemäße Mischung enthaltende Aroma- und Riechstoffmischungen zeichnen sich durch eine hohe Substantivität aus. Dieser Effekt zeigt sich insbesondere auf Substraten wie Haut, Haar und textilen Fasern (z.B. Wolle, Baumwolle, Leinen, synthetische Fasern).

Dieser Effekt wird weiter unten im Rahmen der anwendungstechnischen Beispiele näher erläutert.

Die Produkte, in denen die Tetrahydrofuranderivate der Formel (I), erfindungsgemäße Mischungen davon und erfindungsgemäße Aroma- und Riechstoffmischungen einformuliert werden, können weitere Inhaltstoffe, Additive und Substanzen umfassen, welche beispielsweise ausgewählt sind aus: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweißhemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Polyole, Tenside, Elektrolyte, organische Lösungsmittel oder Silikonderivate.

Einige der weiteren Inhaltstoffe, Additive und Substanzen werden im Folgenden näher erläutert:

### KOSMETISCHE INHALTSTOFFE, ADDITIVE UND SUBSTANZEN

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia® SP von Cognis;
- Polyalkylenglycole sowie
- Glycerincarbonat.

Im Folgenden werden besonders geeignete Emulgatoren näher erläutert:
**Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.
**Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.
**Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.
**Sorbitanester.** Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.
**Polyglycerinester.** Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.
**Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.
**Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Kühlstoffe

Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

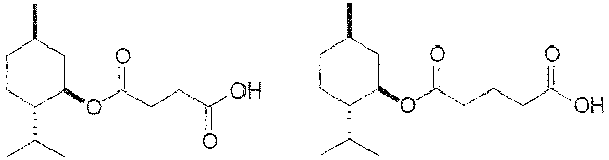

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat® MGC), Menthol Propylenglycol Carbonate (FEMA GRAS 3784 = Frescolat® MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat® ML) und insbesondere das Menthone Glyceryl Acetal (FEMA GRAS 3807) bzw. Menthone Glyceryl Ketal (FEMA GRAS 3808), das unter der Bezeichnung Frescolat® MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat® MGA, Frescolat® ML, Frecolat® MGC und Frescolat® MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäurean-hydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Silikonverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfaktoren

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan® AP)
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 2-(4-Diethylamino-2-hydroxybenzoyl)-benzoic acid hexylester (Uvinul® A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000, Eusolex® T, Eusolex® T-ECO, Eusolex® T-S, Eusolex® T-Aqua, Eusolex® T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE® oder Z-COTE HP1® verwendet.

### Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. VitaminE-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

**Keimhemmende Mittel.** Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

**Enzyminhibitoren.** Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder - phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

**Geruchsabsorber.** Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien.** Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichloro-hydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

### Insektenrepellentien

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Inhaltsstoffe für Mund- und Zahnpflegemittel

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

### Hydrotrope

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, ß-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### INHALTSTOFFE, ADDITIVE UND SUBSTANZEN FÜR WASCH- UND REINIGUNGSMITTEL

### Tenside

Als Tenside zur Herstellung der Wasch- oder Reinigungsmittel können neben den nichtionischen Tensiden auch Anion-, Kation-, Ampho- und/oder Niotenside und verzweigte Alkylsulfaten verwendet werden.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C12-14-Alkohole mit 3 EO, 4 EO oder 7 EO, C9-11-Alkohol mit 7 EO, C13-5-Alkohole mit 3 EO, 5 EO, 7 EO oder 8 EO, C12-18-Alkohole mit 3 EO, 5 EO oder 7 EO und Mischungen aus diesen, wie Mischungen aus C12-4-Alkohol mit 3 EO und C12-8-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise, sondern statistisch verteilt sind. Solche Produkte sind durch gleichzeitige Einwirkung von Ethylen- und Propylenoxid auf Fettalkohole erhältlich.

Eine weitere Klasse von nichtionischen Tensiden, die vorteilhaft zur Herstellung von Wasch- oder Reinigungsmitteln eingesetzt werden kann, sind die Alkylpolyglycoside (APG). Einsetzbare Alkypolyglycoside genügen der allgemeinen Formel RO(G)Z, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosidierungsgrad z liegt dabei zwischen 1,0 und 4,0, vorzugsweise zwischen 1,0 und 2,0 und insbesondere zwischen 1,1 und 1,4.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können zur Herstellung der Wasch- oder Reinigungsmittel geeignet sein. Die Menge dieser nichtionischen Tenside beträgt vorzugsweise nicht mehr als die der ethoxylierten Fettalkohole, insbesondere nicht mehr als die Hälfte davon.

### Weitere geeignete Tenside sind Polyhydroxyfettsäureamide der Formel R-CO-N(R1)-[Z],

in der RCO für einen aliphatischen Acylrest mit 6 bis 22 Kohlenstoffatomen, R1 für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Zur Gruppe der Polyhydroxyfettsäureamide gehören auch Verbindungen der Formel R-CO-N(R1-O-R2)- [Z], in der R für einen linearen oder verzweigten Alkyl- oder Alkenylrest mit 7 bis 12 Kohlenstoffatomen, R1 für einen linearen, verzweigten oder cyclischen Alkylrest oder einen Arylrest mit 2 bis 8 Kohlenstoffatomen und R2 für einen linearen, verzweigten oder cyclischen Alkylrest oder einen Arylrest oder einen Oxy-Alkylrest mit 1 bis 8 Kohlenstoffatomen steht, wobei C1-4-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes. [Z] wird vorzugsweise durch reduktive Aminierung eines Zuckers erhalten, beispielsweise Glucose, Fructose, Maltose, Lactose, Galactose, Mannose oder Xylose. Die N-Alkoxy- oder N-Aryloxy-substituierten Verbindungen können dann durch Umsetzung mit Fettsäuremethylestern in Gegenwart eines Alkoxids als Katalysator in die gewünschten Polyhydroxyfettsäureamide überführt werden.

Der Gehalt an nichtionischen Tensiden beträgt den flüssigen Wasch- und Reinigungsmitteln bevorzugt 5 bis 30 Gew.%, vorzugsweise 7 bis 20 Gew.% und insbesondere 9 bis 15 Gew.%, jeweils bezogen auf das gesamte Mittel.

Als anionische Tenside werden beispielsweise solche vom Typ der Sulfonate und Sulfate eingesetzt. Als Tenside vom Sulfonat-Typ kommen dabei vorzugsweise C9-3-Alkylbenzolsulfonate, Olefinsulfonate, d.h. Gemische aus Alken- und Hydroxyalkansulfonaten sowie Disulfonaten, wie man sie beispielsweise aus C12-8-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C12-8-Alkanen beispielsweise durch Sulfochlorierung oder Sulfoxidation mit anschließender Hydrolyse bzw. Neutralisation gewonnen werden. Ebenso sind auch die Ester von alpha-Sulfofettsäuren (Estersulfonate), zum Beispiel die alpha-sulfonierten Methylester der hydrierten Kokos-, Palmkern- oder Talgfettsäuren geeignet.

Auch geeignet sind Sulfonierungsprodukte von ungesättigten Fettsäuren, beispielsweise Ölsäure, in geringen Mengen, vorzugsweise in Mengen nicht oberhalb etwa 2 bis 3 Gew.-%. Insbesondere sind alpha -Sulfofettsäurealkylester bevorzugt, die eine Alkylkette mit nicht mehr als 4 C-Atomen in der Estergruppe aufweisen, beispielsweise Methylester, Ethylester, Propylester und Butylester. Mit besonderem Vorteil werden die Methylester der alpha -Sulfofettsäuren (MES), aber auch deren verseifte Disalze eingesetzt.

Als weitere anionische Tenside kommen Fettsäure-Derivate von Aminosäuren, beispielsweise von N-Methyltaurin (Tauride) und/oder von N-Methylglycin (Sarkoside) in Betracht. Insbesondere bevorzugt sind dabei die Sarkoside bzw. die Sarkosinate und hier vor allem Sarkosinate von höheren und gegebenenfalls einfach oder mehrfach ungesättigten Fettsäuren wie Oleylsarkosinat.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C12-C18-Fettalkohole, beispielsweise aus Kokosfettalkohol, Talgfettalkohol, Lauryl-, Myristyl-, Cetyl- oder Stearylalkohol oder der C10-C20-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C12-C16-Alkylsulfate und C12-C15-Alkylsulfate sowie C14-C15-Alkylsulfate bevorzugt. Auch 2,3-Alkylsulfate, die beispielsweise als Handelsprodukte der Shell Oil Company unter dem Namen DAN(R) erhalten werden können, sind geeignete Aniontenside.

Auch die Schwefelsäuremonoester der mit 1 bis 6 Mol Ethylenoxid ethoxylierten geradkettigen oder verzweigten C7-21-Alkohole, wie 2-Methyl-verzweigte C9-11-Alkohole mit im Durchschnitt 3,5 Mol Ethylenoxid (EO) oder C12-18-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C8-18-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Insbesondere bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di-oder Triethanolamin, vorliegen. Vorzugsweise liegen die anionischen Tenside in Form ihrer Natrium- oder Kaliumsalze, insbesondere in Form der Natriumsalze vor.

Der Gehalt bevorzugter flüssiger Wasch- und Reinigungsmittel an anionischen Tensiden beträgt 1 bis 30 Gew.%, vorzugsweise 4 bis 25 Gew.% und insbesondere 5 bis 22 Gew.%, jeweils bezogen auf das gesamte Mittel. Es ist besonders bevorzugt, dass die Menge an Fettsäureseife mindestens 2 Gew.%und besonders bevorzugt mindestens 3 Gew.%und insbesondere bevorzugt mindestens 4 Gew.%beträgt.

Als weitere Tenside kommen zur Herstellung der erfindungsgemäßen Wasch- oder Reinigungsmittel sogenannte Gemini-Tenside in Betracht. Hierunter werden im Allgemeinen solche Verbindungen verstanden, die zwei hydrophile Gruppen und zwei hydrophobe Gruppen pro Molekül besitzen. Diese Gruppen sind in der Regel durch einen sogenannten "Spacer" voneinander getrennt. Dieser Spacer ist in der Regel eine Kohlenstoffkette, die lang genug sein sollte, dass die hydrophilen Gruppen einen ausreichenden Abstand haben, damit sie unabhängig voneinander agieren können. Derartige Tenside zeichnen sich im Allgemeinen durch eine ungewöhnlich geringe kritische Micellkonzentration und die Fähigkeit, die Oberflächenspannung des Wassers stark zu reduzieren, aus. In Ausnahmefällen werden jedoch unter dem Ausdruck Gemini-Tenside nicht nur dimere, sondern auch trimere Tenside verstanden.

Gemini-Tenside zur Herstellung von Wasch- oder Reinigungsmitteln sind beispielsweise sulfatierte Hydroxymischether gemäß der deutschen Patentanmeldung DE-A-43 21 022 oder Dimeralkoholbis- und Trimeralkohol-tris-sulfate und -ethersulfate gemäß der deutschen Patentanmeldung DE-A- 195 03 061. Endgruppenverschlossene dimere und trimere Mischether gemäß der deutschen Patentanmeldung DE-A-195 13 391 zeichnen sich insbesondere durch ihre Bi- und Multifunktionalität aus. So besitzen die genannten endgruppenverschlossenen Tenside gute Netzeigenschaften und sind dabei schaumarm, so dass sie sich insbesondere für den Einsatz in maschinellen Wasch- oder Reinigungsverfahren eignen.

Bevorzugt sind aus anwendungstechnischer Sicht Mischungen aus anionischen und nichtionischen Tensiden. Der Gesamt-Tensidgehalt des flüssigen Wasch- und Reinigungsmittel liegt vorzugsweise unterhalb von 40 Gew.- % und besonders bevorzugt unterhalb von 35 Gew.%, bezogen auf das gesamte flüssige Wasch- und Reinigungsmittel.

### Gerüststoffe

Als Gerüststoffe bzw. Builder, die in den flüssigen Wasch- und Reinigungsmitteln enthalten sein können, sind insbesondere Silikate, Aluminiumsilikate (insbesondere Zeolithe), Carbonate, organische Co-builder, Phosphate, Salze organischer Di- und Polycarbonsäuren sowie Mischungen dieser Stoffe zu nennen.

Geeignete kristalline, schichtförmige Natriumsilikate besitzen die allgemeine Formel NaMSiₓO₂ₓ₊₁*H2O, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilikate der angegebenen Formel sind solche, in denen M für Natrium steht und x die Werte 2 oder 3 annimmt. Insbesondere sind sowohl beta - als auch delta - Natriumdisilikate Na₂Si₂O₅*yH₂O bevorzugt.

Einsetzbar sind auch amorphe Natriumsilikate mit einem Modul Na₂O: SiO₂ von 1: 2 bis 1: 3,3, vorzugsweise von 1: 2 bis 1: 2,8 und insbesondere von 1: 2 bis 1: 2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, dass die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharte Beugungsmaxima liefern. Dies ist so zu interpretieren, dass die Produkte mikrokristalline Bereiche der Größe 10 bis einige Hundert nm aufweisen, wobei Werte bis maximal 50 nm und insbesondere bis maximal 20 nm bevorzugt sind. Derartige sogenannte röntgenamorphe Silikate, weisen ebenfalls eine Löseverzögerung gegenüber den herkömmlichen Wassergläsern auf. Insbesondere bevorzugt sind verdichtete/kompaktierte amorphe Silikate, compoundierte amorphe Silikate und übertrocknete röntgenamorphe Silikate.

Ein verwendbarer feinkristalliner, synthetischer und gebundenes Wasser enthaltender Zeolith ist vorzugsweise Zeolith A und/oder P. Als Zeolith P wird Zeolith MAP TM (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma SASOL unter dem Markennamen VEGOBOND AX(R) vertrieben wird und durch die Formel

nNa2O*(1-n)K₂O*Al₂O₃.(2-2,5)*SiO₂.(3,5-5,5)*H₂O,

beschrieben werden kann

entspricht. Der Zeolith kann als sprühgetrocknetes Pulver oder auch als ungetrocknete, von ihrer Herstellung noch feuchte, stabilisierte Suspension zum Einsatz kommen. Für den Fall, dass der Zeolith als Suspension eingesetzt wird, kann diese geringe Zusätze an nichtionischen Tensiden als Stabilisatoren enthalten, beispielsweise 1 bis 3 Gew.%, bezogen auf Zeolith, an ethoxylierten C₁₂-C₁₈-Fettalkoholen mit 2 bis 5 Ethylenoxidgruppen, C₁₂-C₁₄-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Messmethode: Coulter Counter) auf und enthalten vorzugsweise 18 bis 22 Gew.%, insbesondere 20 bis 22 Gew.% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate.

Als Builder sind organische Co-builder geeignet, insbesondere Polycarboxylate/Polycarbonsäuren, polymere Polycarboxylate, Asparaginsäure, Polyacetale, Dextrine, sowie Phosphonate.

Polymere Polycarboxylate sind beispielsweise die Alkalimetallsalze der Polyacrylsäure oder der Polymethacrylsäure, beispielsweise solche mit einer relativen Molekülmasse von 500 bis 70000 g/mol. Bei den für polymere Polycarboxylate angegebenen Molmassen handelt es sich im Sinne dieser Schrift um gewichtsmittlere Molmassen Mw der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsaeure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2.000 bis 70.000 g/mol, vorzugsweise 20.000 bis 50.000 g/mol und insbesondere 30.000 bis 40.000 g/mol.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder die als Monomere Salze der Acrylsäure und der 2-Alkylallylsulfonsaeure sowie Zucker-Derivate enthalten.

Weitere bevorzugte Copolymere sind solche, die als Monomere vorzugsweise Acrolein und Acrylsäure/Acrylsäuresalze bzw. Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, die neben Co-builder-Eigenschaften auch eine bleichstabilisierende Wirkung aufweisen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500.000 g/mol. Dabei ist ein Polysaccharid mit einem Dextrose- äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weissdextrine mit höheren Molmassen im Bereich von 2000 bis 30000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Ein an C6 des Saccharidrings oxidiertes Produkt kann besonders vorteilhaft sein.

Ein bevorzugtes Dextrin ist in der britischen Patentanmeldung GB 9,419,091 B1 beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Derartige oxidierte Dextrine und Verfahren ihrer Herstellung sind beispielsweise aus den europäischen Patentanmeldungen EP 032202 A**,** EP 0427349 A**,** EP 0472042 A und EP 0542496 A sowie den internationalen Patentanmeldungen WO 1992/018542 A**,** WO 1993/008251 A**,** WO 1994/ 028030 A**,** WO 1995/007303 A**,** WO 1995/012619 A und WO 1995/020608 A bekannt. Ein an C₆ des Saccharidrings oxidiertes Produkt kann besonders vorteilhaft sein.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate, wie sie beispielsweise in den US-amerikanischen Patentschriften US 4,524,009**,** US 4,639 325**,** in der europäischen Patentanmeldung EP 0150930 A und der japanischen Patentanmeldung JP 1993/339896 A beschrieben werden.

Weitere brauchbare organische Co-builder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten. Derartige Co-builder werden beispielsweise in der internationalen Patentanmeldung WO 1995/020029 A beschrieben.

Eine weitere Substanzklasse mit Co-buildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Co-builder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Wasch- und Reinigungsmittel auch Bleiche enthalten, bevorzugt sein Aminoalkan-phosphonate, insbesondere DTPMP, einzusetzen, oder Mischungen aus den genannten Phosphonaten zur Herstellung der Mittel zu verwenden.

Darüber hinaus können alle Verbindungen, die in der Lage sind, Komplexe mit Erdalkaliionen auszubilden, als Co-builder eingesetzt werden.

Weitere brauchbare organische Gerüstsubstanzen sind auch die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von Wasch- und/oder Reinigungsmitteln. Insbesondere sind hierbei Citronensäure, Bernsteinsäure, Glutarsäure, Adipinsäure, Gluconsäure und beliebige Mischungen aus diesen zu nennen.

### Bleichmittel und Bleichkatalysatoren

Unter den als Bleichmitteln dienenden, in Wasser H2O2 liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie H2O2 liefernde persaure Salze oder Persäuren, wie Perbenzoate, Peroxophthalate, Diperazelainsäure, Phthaloiminopersäure oder Diperdodecandisäure. Um beim Waschen bei Temperaturen von 60 °C und darunter eine verbesserte Bleichwirkung zu erreichen, können Bleichaktivatoren in die Wasch- und Reinigungsmittel eingearbeitet werden. Als Bleichaktivatoren können Verbindungen, die unter Perhydrolysebedingungen aliphatische Peroxocarbonsäuren mit vorzugsweise 1bis 10 C-Atomen, insbesondere 2 bis 4 C-Atomen, und/oder gegebenenfalls substituierte Perbenzoesäure ergeben, eingesetzt werden. Geeignet sind Substanzen, die O-und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin(DADHT), acylierte Glykolurile, insbesondere Tetraacetylglykoluril (TAGU), N-Acylimide,insbesondere N-Nonanoylsuccinimid (NOSI), acylierte Phenolsulfonate, insbesonderen-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran. Zusätzlich zu den konventionellen Bleichaktivatoren oder an deren Stelle können auch sogenannte Bleichkatalysatoren in die Textilbehandlungsmitteleingearbeitet werden. Bei diesen Stoffen handelt es sich um bleichverstärkende Übergangsmetallsalze bzw. Übergangsmetallkomplexe wie beispielsweise Mn-,Fe-, Co-, Ru- oder Mo-Salenkomplexe oder -carbonylkomplexe. Auch Mn-, Fe-, Co-,Ru-, Mo-, Ti-, V- und Cu-Komplexe mit stickstoffhaltigen Tripod-Liganden sowie Co-, Fe-, Cu- und Ru-Amminkomplexe sind als Bleichkatalysatoren verwendbar.

### Verdickungsmittel

Ein flüssiges Wasch- und Reinigungsmittel kann ein Verdickungsmittelenthalten. Das Verdickungsmittel kann beispielsweise einen Polyacrylat-Verdicker, Xanthan Gum, Gellan Gum, Guarkernmehl, Alginat, Carrageenan, Carboxymethylcellulose, Bentonite, Wellan Gum, Johannisbrotkernmehl, Agar-Agar, Tragant, Gummi arabicum, Pektine, Polyosen, Stärke, Dextrine, Gelatine und Casein umfassen. Aber auch abgewandelte Naturstoffe wie modifizierten Stärken und Cellulosen, beispielhaftseien hier Carboxymethylcellulose und andere Celluloseether, Hydroxyethyl- und -propylcellulose sowie Kernmehlether genannt, können als Verdickungsmittel eingesetzt werden.

Zu den Polyacryl- und Polymethacryl-Verdickern zählen beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung gemäß "International Dictionary of Cosmetic Ingredients "der "The Cosmetic, Toiletry and Fragrance Association (CTFA)": Carbomer),die auch als Carboxyvinylpolymere bezeichnet werden. Solche Polyacrylsäuren sind u.a. von der Fa. 3V Sigma unter dem Handelsnamen Polygel®,z.B. Polygel DA, und von der Fa. B.F. Goodrich unter dem Handelsnamen Carbopol® erhältlich, z.B. Carbopol 940 (Molekulargewicht ca. 4.000.000), Carbopol 941 (Molekulargewicht ca. 1.250.000) oder Carbopol 934 (Molekulargewicht ca. 3.000.000).Weiterhin fallen darunter folgende Acrylsäure-Copolymere: (i) Copolymere von zwei oder mehr Monomeren aus der Gruppe der Acrylsäure, Methacrylsäure und ihrer einfachen, vorzugsweise mit C1-4-Alkanolen gebildeten, Ester (INCI AcrylatesCopolymer), zu denen etwa die Copolymere von Methacrylsäure, Butylacrylat und Methylmethacrylat (CAS-Bezeichnung gemäß Chemical Abstracts Service: 25035-69-2)oder von Butylacrylat und Methylmethacrylat (CAS 25852-37-3) gehören und die beispielsweise von der Fa. Rohm and Haas unter den Handelsnamen Aculyn® und Acusol® sowie von der Firma Degussa (Goldschmidt) unter dem Handelsnamen Tego® Polymer erhältlich sind, z.B. die anionischen nicht assoziativen Polymere Aculyn 22, Aculyn 28, Aculyn 33 (vernetzt), Acusol 810,Acusol 820, Acusol 823 und Acusol 830 (CAS 25852-37-3); (ii) vernetzte hochmolekulare Acrylsäurecopolymere, zu denen etwa die mit einem Allylether der Saccharose oder des Pentaerythrits vernetzten Copolymere von C10-30-Alkylacrylaten mit einem oder mehreren Monomeren aus der Gruppe der Acrylsäure, Methacrylsäure und ihrer einfachen, vorzugsweise mit C1-4-Alkanolen gebildeten, Ester (IN-Cl Acrylates/C10-30Alkyl Acrylate Crosspolymer) gehören und die beispielsweise von der Fa. B.F. Goodrich unter dem Handelsnamen Carbopol® erhältlich sind, z.B. das hydrophobierte Carbopol ETD 2623 und Carbopol 1382 (INCI Acrylates/C10-30Alkyl Acrylate Crosspolymer) sowie Carbopol Aqua 30 (früher Carbopol EX 473).

Ein weiteres bevorzugt einzusetzendes polymeres Verdickungsmittel ist Xanthan Gum, ein mikrobielles anionisches Heteropolysaccharid, das von Xanthomonascampestris und einigen anderen Species unter aeroben Bedingungen produziert wird und eine Molmasse von 2 bis 15 Millionen Dalton aufweist. Xanthan wird aus einer Kette mit beta-1,4-gebundener Glucose (Cellulose) mit Seitenketten gebildet. Die Struktur der Untergruppen besteht aus Glucose, Mannose, Glucuronsaeure,Acetat und Pyruvat, wobei die Anzahl der Pyruvat-Einheiten die Viskosität des Xanthan Gums bestimmt. Als Verdickungsmittel kommt insbesondere auch ein Fettalkohol in Frage. Fettalkohole können verzweigt oder nichtverzweigt sowie nativen Ursprungs oderpetrochemischen Ursprungs sein. Bevorzugte Fettalkohole haben eine C-Kettenlänge von 10 bis 20 C-Atomen, bevorzugt 12 bis 18. Bevorzugt werden Mischungen unterschiedlicher C-Kettenlängen, wie Talgfettalkohol oder Kokosfettalkohol, eingesetzt. Beispiele sind Lorol® Spezial (C12-14-ROH) oder Lorol® Technisch(C12-18-ROH) (beide ex Cognis). Bevorzugte flüssige Wasch- und Reinigungsmittel enthalten bezogen auf das gesamte Mittel 0.01 bis 3 Gew.% und vorzugsweise 0.1 bis1 Gew.% Verdickungsmittel. Die Menge an eingesetztem Verdickungsmittel ist dabei abhängig von der Art des Verdickungsmittels und dem gewünschten Grad der Verdickung.

### Enzyme

Die Wasch- und Reinigungsmitteln können Enzyme in verkapselter Form und/oder direkt in den Wasch- und Reinigungsmittel enthalten. Als Enzyme kommen insbesondere solche aus der Klassen der Hydrolasen wie der Proteasen, Esterasen, Lipasen bzw. lipolytisch wirkende Enzyme, Amylasen, Cellulasen bzw. andere Glykosylhydrolasen, Hemicellulase, Cutinasen, beta-Glucanasen, Oxidasen, Peroxidasen, Perhydrolasen und/oder Laccasen und Gemische der genannten Enzyme in Frage. Alle diese Hydrolasen tragen in der Wäsche zur Entfernung von Verfleckungen wie Protein-, fett- oder stärkehaltigen Verfleckungen und Vergrauungen bei. Cellulasen und andere Glykosylhydrolasenkoennen darüber hinaus durch das Entfernen von Pilling und Mikrofibrillen zur Farberhaltung und zur Erhöhung der Weichheit des Textils beitragen. Zur Bleiche bzw. zur Hemmung der Farbübertragung können auch Oxireduktasen eingesetzt werden. Besonders gut geeignet sind aus Bakterienstämmen oder Pilzen wie Bacillus subtilis, Bacillus licheniformis, Streptomyceus griseus und Humicolainsolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere alpha-Amylasen, Iso-Amylasen, Pullulanasen und Pektinasen. Als Cellulasen werden vorzugsweise Cellobiohydrolasen, Endoglucanasen und p-Glucosidasen, die auch Cellobiasen genannt werden, bzw. Mischungen aus diesen eingesetzt. Da sich verschiedene Cellulase-Typen durch ihre CMCase- und Avicelase-Aktivitäten unterscheiden, können durch gezielte Mischungen der Cellulasen die gewünschten Aktivitäten eingestellt werden.

Die Enzyme können an Trägerstoffe adsorbiert sein, um sie gegen vorzeitige Zersetzung zu schützen. Der Anteil der Enzyme, der Enzymflüssigformulierung(en) oder der Enzymgranulate direkt in Wasch- und Reinigungsmittel kann beispielsweise etwa 0,01 bis 5 Gew.%, vorzugsweise 0.12 bis etwa 2.5 Gew.% betragen.

Es kann, beispielsweise bei speziellen Wasch- und Reinigungsmitteln für Konsumenten mit Allergien, aber auch bevorzugt sein, dass das Wasch- und Reinigungsmittel keine Enzyme enthält.

### Elektrolyte

Als Elektrolyte aus der Gruppe der anorganischen Salze kann eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ in den Wasch- und Reinigungsmitteln bevorzugt. Der Anteil an Elektrolyten in den Wasch- und Reinigungsmittel beträgt üblicherweise 0,1 bis 5 Gew.-%.

### Lösungsmittel

Nichtwässrige Lösungsmittel, die in den flüssigen Wasch- und Reinigungsmittel eingesetzt werden können, stammen beispielsweise aus der Gruppe der ein- oder mehrwertigen Alkohole, Alkanolamine oder Glykolether, sofern sie im angegebenen Konzentrationsbereich mit Wasser mischbar sind. Vorzugsweise werden die Lösungsmittel ausgewählt aus Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethyl-, -ethyl-oder -propylether, Dipropylenglykolmonomethyl- oder -ethylether, Di-isopropylenglykolmonomethyl-oder -ethylether, Methoxy-, Ethoxy- oder Butoxytriglykol, 1-Butoxyethoxy-2-propanol,3-Methyl-3-methoxybutanol, Propylen-glykol-t-butylether sowie Mischungen dieser Lösungsmittel. Nichtwässrige Lösungsmittel können in den flüssigen Wasch- und Reinigungsmitteln in Mengen zwischen 0,5 und 15 Gew.%, bevorzugt aber unter 12 Gew.% und insbesondere unterhalb von 9 Gew.% eingesetzt werden.

### Viskosität

Die Viskosität der Wasch- und Reinigungsmittel in flüssiger Form kann mit üblichen Standardmethoden (beispielsweise Brookfield-Viskosimeter LVT-II bei 20 U/min und 20°C, Spindel 3) gemessen werden und liegt für flüssige Waschmittel vorzugsweise im Bereich von 500 bis 5000 mPas. Bevorzugte weisen flüssige Wasch- und Reinigungsmitteln Viskositäten von 700 bis 4000 mPas auf, wobei Werte zwischen 1000 und 3000 mPas besonders bevorzugt sind. Die Viskosität von Weichspülern beträgt vorzugsweise 20 bis 4000 mPas, wobei Werte zwischen 40 und 2000 mPas besonders bevorzugt sind. Insbesondere bevorzugt liegt die Viskosität von Weichspülern von 40 bis 1000 mPas.

### pH-Stellmittel

Um den pH-Wert der flüssigen Wasch- und Reinigungsmitteln in den gewünschten Bereich zu bringen, kann der Einsatz von pH-Stellmitteln angezeigt sein. Einsetzbar sind hier sämtliche bekannten Säuren bzw. Laugen, sofern sich ihr Einsatz nicht aus anwendungstechnischen oder ökologischen Gründen bzw. aus Gründen des Verbraucherschutzes verbietet. Üblicherweise überschreitet die Menge dieser Stellmittel 7 Gew.% der Gesamtformulierung nicht. Der pH-Wert von flüssigen Wasch- und Reinigungsmitteln liegt bevorzugt zwischen 4 und 10 und bevorzugt zwischen 5.5 und 8.5. Der pH-Wert von flüssigen Weichspülern liegt bevorzugt zwischen 1 und 6 und bevorzugt zwischen 1.5 und 3.5.

### Farbstoffe

Um den ästhetischen Eindruck der Textilbehandlungsmittel zu verbessern, können sie mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Wasch- und Reinigungsmittel und gegen Licht sowie keine ausgeprägte Substantivität gegenüber Textilfasern, um diese nicht anzufärben.

### Antiredepositionsmittel

Geeignete Soil-Release-Polymere, die auch als "Antiredepositionsmittel" bezeichnet werden, sind beispielsweise nichtionische Celluloseether wie Methylcellulose und Methylhydroxypropylcellulose mit einem Anteil an Methoxygruppen von 15 bis 30Gew.% und an Hydroxypropylgruppen von 1 bis 15 Gew.%, jeweils bezogen auf den nichtionischen Celluloseether sowie die aus dem Stand der Technik bekannten Polymere der Phthalsäure und/oder Terephthalsäure bzw. von deren Derivaten, insbesondere Polymere aus Ethylenterephthalaten und/oder Polyethylen- und/oder Polypropylenglykolterephthalaten oder anionisch und/oder nichtionisch modifizierten Derivaten von diesen. Geeignete Derivate umfassen die sulfonierten Derivate der Phthalsäure- und Terephthalsäure-Polymere.

### Optische Aufheller

Optische Aufheller (sog. "Weisstöner") können den Wasch- und Reinigungsmitteln zugesetzt werden, um Vergrauungen und Vergilbungen der behandelten Textilen Flächengebilden zu beseitigen. Diese Stoffe ziehen auf die Faser auf und bewirken eine Aufhellung und vorgetäuschte Bleichwirkung, indem sie unsichtbare Ultraviolettstrahlung in sichtbares längenwelliges Lichtumwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiss ergibt. Geeignete Verbindungen stammen beispielsweiseaus den Substanzklassen der 4,4'-Diamino-2,2'-stilbendisulfonsaeuren (Flavonsäuren), 4,4'-Distyryl-biphenylen, Methylumbelliferone, Cumarine, Dihydrochinolinone, 1,3-Diarylpyrazoline,Naphthalsaeureimide, Benzoxazol-, Benzisoxazol- und Benzimidazol-Systeme sowie der durch Heterocyclen substituierten Pyrenderivate. Die optischen Aufheller werden üblicherweise in Mengen zwischen 0 % und 0.3 Gew.%, bezogen auf das fertige Wasch- und Reinigungsmittel, eingesetzt.

### Vergrauungsinhibitoren

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Naturgeeignet, beispielsweise Leim, Gelatine, Salze von Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, zum Beispiel abgebaute Stärke, Aldehydstärken usw. Auch Polyvinylpyrrolidon ist brauchbar. Bevorzugt werden jedoch Celluloseether wie Carboxymethylcellulose (Na-Salz), Methylcellulose, Hydroxyalkylcellulose und Mischether wie Methylhydroxyethylcellulose, Methylhydroxypropylcellulose, Methylcarboxymethyl-cellulose und deren Gemische in Mengen von 0.1 bis 5 Gew.%, bezogen auf die Wasch- und Reinigungsmitteln, eingesetzt.

### Knitterschutzmittel

Da textile Flächengebilde, insbesondere aus Reyon, Zellwolle, Baumwolle und deren Mischungen, zum Knittern neigen können, weil die Einzelfasern gegen Durchbiegen, Knicken, Pressen und Quetschen quer zur Faserrichtung empfindlich sind, können die Wasch- und Reinigungsmittel synthetische Knitterschutzmittel enthalten. Hierzu zählen beispielsweise synthetische Produkte auf der Basis von Fettsäuren, Fettsäureestern, Fettsäureamiden, - alkylolestern,-alkylolamiden oder Fettalkoholen, die meist mit Ethylenoxid umgesetzt sind, oder Produkte auf der Basis von Lecithin oder modifizierter Phosphorsäureester.

### Antimikrobielle Wirkstoffe

Zur Bekämpfung von Mikroorganismen können die Wasch- und Reinigungsmitteln antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppensind beispielsweise Benzalkoniumchloride, Alkylarylsulfonate, Halogenphenole und Phenolmercuriacetat, wobei bei den erfindungemäßen Wasch- und Reinigungsmitteln auch gänzlich auf diese Verbindungen verzichtet werden kann.

### Konservierungsmittel

Die erfindungsgemäßen Wasch- und Reinigungsmittel können Konservierungsmittel enthalten, wobei vorzugsweise nur solche eingesetzt werden, die kein oder nur ein geringes hautsensibilisierendes Potential besitzen. Beispiele sind Sorbinsäure und seine Salze, Benzoesäure und seine Salze, Salicylsäure und seine Salze, Phenoxyethanol, 3-lodo-2-propynylbutylcarbamat, Natrium N-(hydroxymethyl)glycinat, Biphenyl-2-ol sowie Mischungen davon. Ein geeignetes Konservierungsmittel stellt die lösungsmittelfreie, wässrige Kombination von Diazolidinylharnstoff, Natriumbenzoat und Kaliumsorbat (erhältlich als Euxyl® K 500ex Schülke and Mayr) dar, welches in einem pH-Bereich bis 7 eingesetzt werden kann. Insbesondere eignen sich Konservierungsmittel auf Basis von organischen Säuren und/oder deren Salzen zur Konservierung der erfindungsgemäßen, hautfreundlichen Wasch- und Reinigungsmittel.

### Antioxidantien

Um unerwünschte, durch Sauerstoffeinwirkung und andere oxidative Prozesse verursachte Veränderungen an den Wasch- und Reinigungsmitteln und/oder den behandelten textilen Flächengebilden zu verhindern, können die Wasch- und Reinigungsmittel Antioxidantien enthalten. Zu dieser Verbindungsklasse gehören beispielsweise substituierte Phenole, Hydrochinone, Brenzcatechine und aromatische Amine sowie organische Sulfide, Polysulfide, Dithiocarbamate, Phosphite, Phosphonate und Vitamin E.

### Antistatika

Ein erhöhter Tragekomfort kann aus der zusätzlichen Verwendung von Antistatika resultieren, die den Wasch- und Reinigungsmitteln zusätzlich beigefügt werden. Antistatika vergrößern die Oberflächenleitfähigkeit und ermöglichen damit ein verbessertes Abfließen gebildeter Ladungen. Antistatika sind in der Regel Substanzen mit wenigstens einem hydrophilen Molekuelliganden und geben auf den Oberflächen einen mehr oder minder hygroskopischen Film. Diese zumeist grenzflächenaktiven Antistatika lassen sich in stickstoffhaltige (Amine, Amide, quartäre Ammoniumverbindungen), phosphorhaltige (Phosphorsaeureester) und schwefelhaltige (Alkylsulfonate, Alkylsulfate) Antistatika unterteilen. Lauryl-(bzw. Stearyl-)dimethylbenzylammoniumchloride eignen sich als Antistatika für textile Flächengebilde bzw. als Zusatz zu Wasch- und Reinigungsmitteln, wobei zusätzlich ein Avivageeffekt erzielt wird.

### Schauminhibitoren

Zur Verbesserung der Wiederbenetzbarkeit der behandelten textilen Flächengebilde und zur Erleichterung des Bügelns der behandelten textilen Flächengebilde können in den Textilbehandlungsmitteln beispielsweise Silikonderivate eingesetzt werden. Diese verbessern zusätzlich das Ausspülverhalten der Wasch- und Reinigungsmittel durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H-und/oder Si-Cl-Bindungen aufweisen. Die Viskositäten der bevorzugten Silikone liegen bei 25 °C im Bereich zwischen 100 und 100.000 mPas, wobei die Silikone in Mengen zwischen 0.2 und 5 Gew.%, bezogen auf das gesamte Wasch- und Reinigungsmittel eingesetzt werden können.

### UV-Absorber

Schließlich können die Wasch- und Reinigungsmittel auch UV-Absorber enthalten, die auf die behandelten textilen Flächengebilde aufziehen und die Lichtbeständigkeit der Fasern verbessern. Verbindungen, die diese gewünschten Eigenschaften aufweisen, sind beispielsweise die durch strahlungslose Deaktivierung wirksamen Verbindungen und Derivate des Benzophenons mit Substituenten in 2- und/oder4-Stellung. Weiterhin sind auch substituierte Benzotriazole, in 3-Stellung Phenyl-substituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet.

### Schwermetallkomplexbildner

Um die durch Schwermetalle katalysierte Zersetzung bestimmter Waschmittel-Inhaltsstoffe zu vermeiden, können Stoffe eingesetzt werden, die Schwermetalle komplexieren. Geeignete Schwermetallkomplexbildner sind beispielsweise die Alkalisalze der Ethylendiamintetraessigsäure(EDTA) oder der Nitrilotriessigsäure (NTA) sowie Alkalimetallsalze von anionischen Polyelektrolyten wie Polymaleaten und Polysulfonaten. Eine bevorzugte Klasse von Komplexbildnern sind die Phosphonate, die in bevorzugten Textilbehandlungsmitteln in Mengen von 0.01 bis 2.5 Gew.-%, vorzugsweise 0.02 bis 2 Gew.% und insbesondere von 0.03 bis 1.5 Gew.% enthalten sind. Zu diesen bevorzugten Verbindungen zählen insbesondere Organophosphonate wie beispielsweise1-Hydroxyethan-1,1-diphosphonsaeure (HEDP), Aminotri(methylenphosphonsäure) (ATMP), Diethylentriamin-penta (methylenphosphonsäure) (DTPMP bzw. DETPMP) sowie 2-Phosphonobutan-1,2,4-tricarbonsäure (PBS-AM), die zumeist in Form ihrer Ammonium- oder Alkalimetallsalze eingesetzt werden.

### GEWERBLICHE ANWENDBARKEIT

Vorzugsweise werden die Tetrahydrofuranderivate der Formel (I) der vorliegenden Erfindung oder Mischungen davon in Aroma- und Riechstoffmischungen eingesetzt, welche vorzugsweise wiederum in parfümierten Produkten einformuliert werden können.

Besonders bevorzugte erfindungsgemäße parfümierte Produkte sind Wasch- und Reinigungsmittel, Hygiene- oder Pflegeprodukte, insbesondere im Bereich der Körper- und Haarpflege, der Kosmetik und des Haushalts.

Demgemäß ist ein Aspekt der vorliegenden Erfindung kosmetische Produkte, umfassend
(a) ein oder mehrere Tetrahydrofuranderivate der Formel (I) oder
(b) Aroma- oder Riechstoffmischungen, umfassend
   (i) ein oder mehrere Tetrahydrofuranderivate der Formel (I),
   (ii) einen oder mehrere weitere Riech- oder Aromastoffe ,
   mit der Maßgabe, dass die Gesamtmenge an enthaltenen Tetrahydrofuranderivate bezogen auf die gesamte Aroma- oder Riechstoffmischung im Bereich von 0,00001 bis 40 Gew.-%, bevorzugt von 0,1 bis 15 Gew.-%, besonders bevorzugt von 0,2 bis 10 Gew.% liegt und die Einsatzmenge der Aroma- oder Riechstoffmischung (i+ii) bezogen auf die kosmetischen Produkte 2 bis 6 Gew.-% beträgt.

Ein weiterer Aspekt der vorliegenden Erfindung sind daher auch Haushaltsprodukte, umfassend
(a) ein oder mehrere Tetrahydrofuranderivate der Formel (I) oder
(b) Aroma- oder Riechstoffmischungen, umfassend
   (i) ein oder mehrere Tetrahydrofuranderivate der Formel (I),
   (ii) einen oder mehrere weitere Riech- oder Aromastoffe,
   mit der Maßgabe, dass die Gesamtmenge an enthaltenen Tetrahydrofuranderivate der Formel (I) bezogen auf die gesamte Aroma- oder Riechstoffmischung im Bereich von 0,00001 bis 40 Gew.-%, bevorzugt von 0,1 bis 15 Gew.-%, besonders bevorzugt von 0,2 bis 10 Gew.% liegt und die Einsatzmenge der Aroma- oder Riechstoffmischung (i+ii) bezogen auf die kosmetischen Produkte 2 bis 6 Gew.-% beträgt.

Vorzugsweise ist ein solches Produkt ein schwach saures, alkalisches oder neutrales Reinigungsmittel, das insbesondere aus der Gruppe ausgewählt ist bestehend aus
- Allzweckreinigern, Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln,
- Luftverbesserern in flüssiger, gelartiger oder auf einem festen Träger aufgebrachter Form oder als Aerosolspray,
- Wachsen oder eine Polituren, die insbesondere aus der Gruppe ausgewählt ist bestehend aus Möbelpolituren, Fußbodenwachsen und Schuhcremes,
- oder
- Körperpflegemitteln, das insbesondere aus der Gruppe ausgewählt ist bestehend aus Duschgelen und Shampoos.

Eine erfindungsgemäße Riechstoffmischung wird erfindungsgemäß hergestellt, indem die erfindungsgemäßen Verbindung oder eine wie oben definierte erfindungsgemäße Mischung mit dem oder den weiteren Riechstoffen sowie gegebenenfalls weiteren Bestandteilen der Riechstoffmischung vermischt werden.

Gemäß einer bevorzugten Ausführungsform wird eine erfindungsgemäße Riechstoffmischung wie oben beschrieben hergestellt, wobei die erfindungsgemäßen Verbindungen in einer Menge eingesetzt wird, die ausreicht, um in der Riechstoffmischung eine oder vorzugsweise beide der Geruchsnoten Rum, Whisky, Kaffee zu vermitteln, zu modifizieren und/oder zu verstärken.

Für die neben den erfindungsgemäßen Verbindungen bevorzugt auszuwählenden weiteren Bestandteile bzw. Riechstoffe gilt das weiter oben Gesagte entsprechend.

### BEISPIELE

Die folgenden Beispiele erläutern die Erfindung, sofern nicht anders angegeben beziehen sich Anteile und Prozente auf das Gewicht.

Verwendete Abkürzungen:

Dipropylenglycol (DPG), Diethylphthalat (DEP), Triethylcitrat (TEC).

Für Erläuterungen der Produktnamen der Riechstoffe siehe z.B. S. Arctander, Perfume and Flavor Materials, Vol. I und II, Montclair, N. J., 1969, Selbstverlag oder H. Surburg, J. Panten, "Common Fragrance and Flavor Materials", 5th. Ed., Wiley-VCH, Weinheim 2006.

### Herstellungsbeispiel: Hydrierung von Furan-2-carbonsäureethylester

3010g (21,5 mol) Furan-2-carbonsäureethylester, 500ml Ethanol (1% MEK) und 20g Katalysator, Palladium auf A-Kohle trocken, 5% Pd, Typ K-0227T) werden zusammen vorgelegt und unter 20bar Wasserstoffdruck aufgeheizt. Die Reaktion setzt bei 30-40°C ein und ist relativ stark exotherm. Nach 1-1,5h bei 60-70°C ist die Wasserstoffaufnahme so gut wie beendet. Es wird langsam auf 140-150°C aufgeheizt und noch 2-3h bei dieser Temperatur nachgerührt. GC-Kontrolle: kein Edukt mehr vorhanden.

### Vom Katalysator abfiltrieren und das Filtrat am Rotationsverdampfer einengen (Wasserbad: 60-70°C, 200-20mbar). Die Rohausbeute beträgt 3055g

Das eingedampfte Rohprodukt wird unter Zusatz von 0,1Gew.-% Na2CO3 an einer kurzen Kolonne destilliert (Kp.: 91-93°C/40mbar).

Ausbeute: 2999g (96,9%d.Th.)

**¹H NMR** (400 MHz, Chloroform-d) δ 4.44 (dd, J = 8.4, 5.2 Hz, 1H), 4.20 (qd, J = 7.1, 1.7 Hz, 2H), 4.05 -3.98 (m, 1H), 3.95 -3.88 (m, 1H), 2.30 - 2.19 (m, 1H), 2.06 -1.85 (m, 3H), 1.29 (t, J =7.1 Hz, 3H) **¹³C NMR** (101 MHz, CDCl3) δ 173.38, 76.80, 69.33, 60.87, 30.20, 25.27, 14.24

### BEISPIELE 1-3: Parfümölmischungen

### Parfümölmischung 1:

| **BESTANDTEIL** | **MENGE** |
|---|---|
| PROPYLENGLYKOL-1,2 | 203,9 |
| HEXYLZIMTALDEHYD ALPHA | 95,0 |
| TETRAHYDROLINALOOL | 63,5 |
| LILIAL | 60,0 |
| HEXYLSALICYLAT | 57,5 |
| ISO E SUPER | 51,5 |
| AMYLSALICYLAT | 44,0 |
| BENZYLACETAT | 40,0 |
| ORANGE OIL SWEET EXTRA | 27,5 |
| HERBYLPROPIONAT | 26,0 |
| TERPINEOL REIN | 23,0 |
| GLOBALIDE® | 22,5 |
| HEDION | 21,0 |
| TUBEREUSE BASE | 15,0 |
| PHENOXANOL | 12,0 |
| AGRUMEX LC | 11,5 |
| ALDEHYD C18 SOG. | 11,5 |
| CITRONELLOL 950 | 11,0 |
| INTRELEVENALDEHYD SPEZ. 1% DPG | 10,0 |
| GERANIOL SUPRA | 9,5 |
| METHYLANTHRANILAT | 8,0 |
| APRICONIA BASE 1% DPG | 7,5 |
| AURANTIOL | 7,5 |
| BENZYLPROPIONAT | 7,5 |
| HELIONAL | 7,5 |
| UNDECAVERTOL/ GIV | 7,5 |
| EUGENOL NAT. | 7,3 |
| STYROLYLACETAT | 7,0 |
| BENZYLALKOHOL DD | 6,5 |
| HELIOTROPIN/PIPERONAL | 5,8 |
| LINALOOL | 5,0 |
| ISOEUGENOL | 4,5 |
| PATCHOULI LIGHT | 4,5 |
| LIVESCONE 10% DPG | 4,0 |
| METHYLBUTENOL-3,2 10% DPG | 4,0 |
| HERBAFLORAT | 3,6 |
| YLANG BAS | 3,5 |
| HEXENOL CIS-3 | 3,3 |
| ALDEHYD C11 UNDECYLENIC | 3,0 |
| DYNASCONE 10% DPG | 3,0 |
| HYACINTHE BODY 10% DPG | 3,0 |
| JAVANOL | 3,0 |
| LINALYLACETAT | 3,0 |
| METHYLBENZOAT | 3,0 |
| ALDEHYD C11 ISO 10% DPG | 2,8 |
| CITRONELLYLACETAT EXTRA | 2,8 |
| ALDEHYD C12 LAURIN | 2,5 |
| KRESOLMETHYLETHER P(CR< 10 PPM) | 2,5 |
| AMBRETTOLIDE | 2,4 |
| FRESCOMENTHE | 2,2 |
| GERANIUMOEL BOURBON | 2,2 |
| PHENYLETHYLMETHYLETHER | 2,2 |
| FLORHYDRAL 10% DPG | 2,0 |
| NEROL 900 | 2,0 |
| PERUBALSAMOEL ED | 2,0 |
| PHENOXYETHYLALKOHOL 10% DPG | 2,0 |
| ALDEHYD C14 SOG | 1,7 |
| NEROLIN BROMELIA | 1,5 |
| ORRIS BUTTER TYPE BASE 10% DPG | 1,5 |
| STEMONE | 1,5 |
| OCTANON-2 | 1,3 |
| ZIMTBLAETTEROEL REKT. | 1,3 |
| BORNEOL L/ISOBORNEOL 65/35 10% DPG | 1,0 |
| CARVON L 10% DPG | 1,0 |
| HEXENYLACETAT CIS-3 | 1,0 |
| ISOEUGENOLMETHYLETHER | 1,0 |
| NELKENBLUETENOEL | 1,0 |
| NEROLI BASE 2 | 1,0 |
| BHT JONOL | 0,9 |
| GERANYLACETAT PUR | 0,9 |
| METHYLNAPHTHYLKETON BETA KRIST. | 0,9 |
| AGRUNITRIL | 0,8 |
| PRENYLACETAT | 0,8 |
| NEROLIDOL | 0,7 |
| PHENYLETHYLALKOHOL | 0,7 |
| BENZYLSALICYLAT | 0,6 |
| INDOL FF | 0,6 |
| METHYLOCTINCARBONAT 1% DPG | 0,5 |

Mit Zusatz von 0,2% der Tetrahydrofuranderivate der Verbindungen 1 bis 9 wird die blumige Kopfnote nicht nur gepuscht sondern verleiht der gesamten Komposition eine zusätzliche Fülle.

### Parfümölmischung 2:

| **BESTANDTEIL** | **MENGE** |
|---|---|
| ALLYLIONON | 2,0 |
| AMAROCIT | 17,0 |
| AMBROCENIDE® 1% DPG | 5,0 |
| AMBROXIDE | 5,0 |
| ATY-12 | 15,0 |
| CEDREN | 90,0 |
| CITRAL FF | 8,0 |
| CUMARIN | 1,0 |
| EVERNYL 10% DPG | 15,0 |
| FILBERTONE 1% DPG | 2,0 |
| GLOBALIDE® | 35,0 |
| HEDION HC/30 | 25,0 |
| HEDION | 21,0 |
| ISO E SUPER | 100,0 |
| ISORALDEIN 95 | 70,0 |
| KEPHALIS | 27,0 |
| LINALOOL | 60,0 |
| LINALYLACETAT | 20,0 |
| LYRAL | 40,0 |
| MAGNOLAN | 4,0 |
| MAJANTOL | 32,0 |
| MUSKATNUSSOEL | 7,0 |
| PALISANDAL | 30,0 |
| PALISANDIN | 30,0 |
| PETITGRAINOEL PARAG | 1,0 |
| PIMENTBLÄTTEROELJAMAICA | 10,0 |
| SANDELWOOD OIL | 4,0 |
| STYROLYLACETAT | 4,0 |
| VERTOFIX FF | 240,0 |
| VETIVEROEL HAITI | 60,0 |
| WACHOLDERBEEROEL RS | 8,0 |

Mit Zusatz von 1% der Tetrahydrofuranderivate Verbindungen 1 bis 9 wird die Mischung frischer, natürlicher und citrischer mit einer interessanten cremigen Kaffee Note.

### Parfümölmischung 3:

| **BESTANDTEIL** | **MENGE** |
|---|---|
| AMBRETTOLIDE | 2,0 |
| BERGAMOTTOEL | 17,0 |
| BENZYLSALICYLAT | 6,0 |
| AMBROXIDE | 2,0 |
| CITRONELLOL | 1,0 |
| CUMARIN | 90,0 |
| DIHYDROMYRCENOL | 22,0 |
| DPG | 33,0 |
| ETHYLENBRASSYLAT | 53,0 |
| ETHYLVANILLIN | 1,0 |
| ETHYLLINALOOL | 12,0 |
| FREESIOL | 20,0 |
| GERANIUMOEL | 5,0 |
| HEDION | 20,0 |
| HELVETOLIDE | 5,0 |
| ISO E SUPER | 36,0 |
| KEPHALIS | 6,0 |
| LAVANDINOEL | 4,0 |
| LILIAL | 7,0 |
| LINALOOL | 25,0 |
| LINALYLACETAT | 7,0 |
| MACROLIDE | 20,0 |
| MUSCENONE | 4,0 |
| ORANGENOEL | 8,0 |
| PATCHOULIOEL | 45,0 |
| PHENYLETHYLPHENYLACETAT | 1,0 |
| STYROLYLACETAT 10% DPG | 5,0 |
| VANILLIN | 9,0 |
| VANILLINISOBUTYRAT | 2,0 |
| VELVIONE | 1,0 |

Mit Zusatz von 2% der Tetrahydrofuranderivate der Verbindungen 1 bis 9 wird die Kopfnote vanilliger und natürlicher weiterhin wird die honigartige Kaffee Note verstärkt.

### Formulierungsbeispiele A bis K

Die Parfümölmischungen 1, 2, und 3 wurden jeweils separat in die nachfolgenden Formulierungen eingearbeitet.

Die oben bei dem jeweiligen Parfümölmischungen beschriebenen geruchlichen Effekte wurden jeweils auch in den nachfolgenden Formulierungen beobachtet.

### Beispiel A: Waschpulver (Mengenangaben als Gew.-%)

| **Material** | **Chemischer Name** | **A1** | **A2** |
|---|---|---|---|
| Natrium Metasilicat Pentahydrat | Sodium Metasilicate Pentahydrate | Ad 100 | Ad 100 |
| Natriumhydrogencarbonat | Sodium hydrogen carbonate | 15,0 | 15,0 |
| Natrium-percarbonat | Sodium carbonate peroxyhydrate | 15,0 | 15,0 |
| Peractive AC Blue | TAED/ Na-Carboxymethylcellulose | 5,00 | 5,00 |
| Genapol OA-080 | Oxoalkohol C14-15, 8EO | 3,00 | 3,00 |
| Texapon K12 Pulver | Sodium Lauryl Sulphate C12 | 7,00 | 7,00 |
| Tinopal CBS-X | | 0,50 | 0,50 |
| Savinase 6.0 T, Type W | Protease | 0,40 | 0,40 |
| Termamyl 120 T | Alpha-Amylase | 0,30 | 0,30 |
| Natriumsulfat | Sodium Sulphate | 5,50 | 5,50 |
| Parfümölmischungen 1, 2, 3 | | 0,30 | 0,50 |

### Beispiel B: Allzweckreiniger (Mengenangaben als Gew.-%)

| **Material** | **Chemischer Name** | **B1** | **B2** |
|---|---|---|---|
| Entionisiertes Wasser | Water | Ad 100 | Ad 100 |
| Mergal K9N | 5-Chloro-2-methyl-3-(2H)-isothiazolone und 2-methyl-3-(2H)-isothiazolone | 0,1 | 0,1 |
| Trinatriumcitrat Dihydrat | Tri Sodium Citrate Dihydrate | 3,0 | 3,0 |
| Zetesol NL-2 | Fatty alcohol C12-14-sulfate, Sodium | 30,0 | 30,0 |
| Imbentin C/125/055 | Fatty alcohol C12-C15, 8EO | 5,0 | 5,0 |
| Ethanol | Ethanol | 2,0 | 2,0 |
| Parfumölmischungen 1,2,3 | | 0,3 | 0,5 |

### Beispiel C: Shampoo (Mengenangaben als Gew.-%

| **Material** | **INCI-Name** | **C1** | **C2** |
|---|---|---|---|
| Entionisiertes Wasser | Water | Ad 100 | Ad 100 |
| Plantacare PS 10 | Sodium Laureth Sulfate, Lauryl Glucoside | 20,0 | 20,0 |
| Euperlan PK 771 | Glycol Distearate, Sodium Lauryl Sulfate, Cocamide MEA, Laureth-10 | 6,0 | 6,0 |
| Dragocid Liquid | Phenoxyethanol, Methylparaben, Ethylparaben, Butylparaben, Propy-Iparaben, Isobutylparaben | 0,5 | 0,5 |
| Natrium Chlorid | Sodium Chloride | 1,4 | 1,4 |
| Citronensäure Monohydrat kristallin | Citric Acid | 0,1 | 0,1 |
| Parfumölmischungen 1, 2, 3 | Parfum (Fragrance) | 0,5 | 0,8 |

### Beispiel D: Duschgel (Mengenangabe als Gew.-%)

| **Material** | **INCI-Name** | **D1** | **D2** |
|---|---|---|---|
| Entionisiertes Wasser | Wasser | Ad 100 | Ad 100 |
| Plantacare PS 10 | Sodium Laureth Sulfate, Lauryl Glucoside | 20,0 | 20,0 |
| Dragocid Liquid | Phenoxyethanol, Methylparaben, Ethylparaben, Butylparaben, Propy-Iparaben, Isobutylparaben | 0,5 | 0,5 |
| Natriumchlorid | Sodium Chloride | 1,4 | 1,4 |
| Citronensäure Monohydrat kristallin | Citric Acid | 1,3 | 1,3 |
| Parfumölmischungen 1, 2 und | Parfum (Fragrance) | 0,5 | 0,7 |

### Beispiel E: Weichspüler (Mengenangaben als Gew.-%)

| **Material** | **Chemischer Name** | **E1** | **E2** |
|---|---|---|---|
| Entionisiertes Wasser | Wasser | Ad 100 | Ad 100 |
| Rewoquat WE 18 | Dialkylesterammonium-ethosulfate | 16,6 | 16,6 |
| Mergal K9N | 5-Chloro-2-methyl-3-(2H)-isothiazolone und 2-methyl-3-(2H)-isothiazolone | 0,10 | 0,10 |
| Dow Corning 1520 Antifoam | Polydimethyl-siloxane | 0,30 | 0,30 |
| Magnesium Chlorid 1%ige Lösung | Magnesium Chlorid Lösung | 10,00 | 10,00 |
| Parfumölmischungen 1, 2, 3 | | 0,55 | 0,75 |

### Beispiel F: Eau de Cologne / Eau de Toilette (Mengenangaben als Gew.-%)

| **Inhaltsstoffe** | **F1** | **F2** |
|---|---|---|
| Parfümölmischungen 1, 2 und 3 | 5 | 10 |
| Ethanol | Ad 100 | Ad 100 |
| Wasser | 18 | 10 |

### Beispiel G: Aerosol-Pumpspray (Mengenangaben als Gew.-%)

| **Inhaltsstoffe** | **G1** | **G2** |
|---|---|---|
| Parfümölmischungen 1, 2 und 3 | 1,0 | 1,5 |
| Ethanol | Ad 100 | Ad 100 |
| Wasser | 5,0 | 8,0 |
| Alpha-Tocopherol | 0,20 | 0,20 |
| Hydroxypropylcellulose | 0,20 | - |
| Rosmarinextrakt, in Ethanol löslich | 0,22 | - |
| Cetylalkohol | 1,00 | 0,50 |

### Beispiel H: Shampoo (Mengenangaben als Gew.-%)

| **Inhaltsstoffe** | **H1** | **H2** | **H3** |
|---|---|---|---|
| Natriumlaurylethersulfat (z.B. Texapon NSO, Fa. Cognis Deutschland GmbH) | 12 | 12 | 12 |
| Cocamidopropylbetain (z.B. Dehyton K, Fa. Cognis Deutschland GmbH) | 2 | 2 | 2 |
| Natriumchlorid | 1,4 | 1,4 | 1,4 |
| Citronensäure | 1,3 | 1,3 | 1,3 |
| Phenoxyethanol, Methyl-, Ethyl-, Butyl- und Propylparaben | 0,5 | 0,5 | 0,5 |
| Parfümölmischungen 1, 2 und 3 | 0,3 | 0,5 | 0,7 |
| Wasser | Ad 100 | Ad 100 | Ad 100 |

### Beispiel I: Waschpulver (Mengenangaben als Gew.-%)

| **Inhaltsstoffe** | **I1** | **I2** | **I3** |
|---|---|---|---|
| Lineares Na-Alkylbenzolsulfonat | 8,8 | 8,8 | 8,8 |
| Ethoxylierter Fettalkohol C12-18 (7 EO) | 4,7 | 4,7 | 4,7 |
| Na-Seife | 3,2 | 3,2 | 3,2 |
| Entschäumer DOW CORNING(R) 2-4248S POWDERED ANTIFOAM, Silikonöl auf Zeolith als Trägermaterial | 3,9 | 3,9 | 3,9 |
| Zeolith 4A | Ad 100 | Ad 100 | Ad 100 |
| Na-Carbonat | 11,6 | 11,6 | 11,6 |
| Na-Salz eines Copolymers aus Acryl- und Maleinsäure (Sokalan CP5) | 2,4 | 2,4 | 2,4 |
| Na-Silicat | 3,0 | 3,0 | 3,0 |
| Carboxymethylcellulose | 1,2 | 1,2 | 1,2 |
| Dequest 2066 ([[(Phosphonome-thyl)imino]bis[(ethylennitrilo)bis (methylen)]]tetrakis-phosphonsäure, Natriumsalz) | 2,8 | 2,8 | 2,8 |
| Optischer Aufheller | 0,2 | 0,2 | 0,2 |
| Na-Sulfat | 6,5 | 6,5 | 6,5 |
| Protease | 0,4 | 0,4 | 0,4 |
| Natriumperborat tetrahydrat | 21,7 | 21,7 | 21,7 |
| Parfümölmischungen 1, 2 und 3 | 0,25 | 0,35 | 0,5 |
| EDTA | 1,0 | 1,0 | 1,0 |

### Beispiel J: Flüssigwaschmittel (Mengenangaben als Gew.-%)

| **Inhaltsstoffe** | **J1** |
|---|---|
| Entionisiertes Wasser | 39,9 |
| Optischer Aufheller | 0,10 |
| Kokos Fettsäuren (C12-C18) | 7,5 |
| Kaliumhydroxid 50%ige Lösung | 4,3 |
| Propan-1,2-diol | 5,00 |
| Fettalkohole C12-C15, 8 EO | 12,0 |
| Na-Salz sekundärer Alkylsulfonate (C13-C17) | 17,0 |
| Triethanolamin | 2,0 |
| Trinatriumcitrat Dihydrat | 5,0 |
| Dequest 2066([[(Phosphonomethyl)imino]bis[(ethylennitrilo)bis (methylen)]]tetrakis-phosphonsäure, Natriumsalz) | 3,0 |
| Ethanol | 3,0 |
| Enzyme | 0,7 |
| Parfümölmischungen 1, 2 und 3 | 0,5 |

### Beispiel K: Flüssigwaschmittel Konzentrat (Mengenangaben als Gew.-%)

| **Inhaltsstoffe** | **K1** |
|---|---|
| Entionisiertes Wasser | 13,4 |
| Kokos Fettsäuren (C12-C18) | 10,0 |
| Fettalkohole C12-C15, 8 EO | 26,0 |
| Na-Salz sekundärer Alkylsulfonate (C13-C17) | 26,5 |
| Triethanolamin | 8,5 |
| Na-Salz von Fettalkoholsulfaten C12-C14 | 3,0 |
| Ethanol | 5,5 |
| Harnstoff | 4,5 |
| Enzyme | 0,9 |
| Citronensäure | 1,0 |
| Parfümölmischungen 1, 2 und 3 | 0,7 |

## Patentansprüche

1. Aroma- und/oder Riechstoffmischungen, umfassend
(i) ein oder mehrere Tetrahydrofuranderivat(e) der Formel (I) 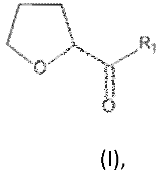
worin R1 ein Wasserstoff, C1-C3- Alkyl oder einen Rest O-R2 darstellt,
worin R2 eine verzweigte oder unverzweigte C1-C5-Alkylgruppe oder verzweigte oder unverzweigte C2-C6-Alkenylgruppe, eine substituierte oder unsubstituierte Cycloalkylgruppe, eine substituierte oder unsubstituierte (C₁-C₈) Alkyl (C₃-C₇) cycloalkylgruppe, eine substituierte oder unsubstituierte Cycloalkenylgruppe, eine substituierte oder unsubstituierte (C₂-C₈) Alkenyl (C₃-C₇) cycloalkylgruppe eine substituierte oder unsubstituierte Arylgruppe oder eine substituierte oder unsubstituierte (C1-C₈) Alkylarylgruppe, eine substituierte oder unsubstituierte (C₂-C₈) Alkenylarylgruppe darstellt,
wobei alle Stereoisomere der Verbindung (I) eingeschlossen sind, und
(ii) einen oder mehrere weitere Riech- oder Aromastoffe,
mit der Maßgabe, dass die Gesamtmenge an enthaltenen Tetrahydrofuranderivaten der Formel (I) bezogen auf die gesamte Aroma- oder Riechstoffmischung im Bereich von 0,00001 bis 40 Gew.-% liegt.

2. Aroma- und/oder Riechstoffmischungen nach Anspruch 1, wobei Tetrahydrofuranderivate der Formel (I), ausgewählt sind aus der Gruppe bestehend aus:
Verbindung 1: Tetrahydrofuran-2-carbonsäuremethylester
Verbindung 2: Tetrahydrofuran-2-carbonsäureethylester
Verbindung 3: Tetrahydrofuran-2-carbonsäurepropylester
Verbindung 4: Tetrahydrofuran-2-carbonsäureallylester
Verbindung 5: Tetrahydrofuran-2-carbonsäure-isopropylester
Verbindung 6: Tetrahydrofuran-2-carbonsäure-2-methylallylester
Verbindung 7: Tetrahydrofuran-2-carbonsäure-isobutylester
Verbindung 8: Tetrahydrofuran-2-carbonsäurebutylester
Verbindung 9: 1-(Tetrahydro-furan-2-yl)-ethanone.

3. Kosmetische Produkte, umfassend
(a) mindestens eine Verbindung der Formel (I), wie in Anspruch 1 oder 2 definiert, oder
(b) Aroma- oder Riechstoffmischungen nach Anspruch 1 oder 2,
mit der Maßgabe, dass die Gesamtmenge an enthaltenen Tetrahydrofuranderivate bezogen auf die gesamte Aroma- oder Riechstoffmischung im Bereich von 0,00001 bis 40 Gew.-% liegt und die Einsatzmenge der Aroma- oder Riechstoffmischung (i+ii) bezogen auf die kosmetischen Produkte 2 bis 6 Gew.-% beträgt.

4. Haushaltsprodukte, umfassend
(a) mindestens eine Verbindung der Formel (I), wie in Anspruch 1 oder 2 definiert, oder
(b) Aroma- oder Riechstoffmischungen nach Anspruch 1 oder 2,
mit der Maßgabe, dass die Gesamtmenge an enthaltenen Tetrahydrofuranderivaten bezogen auf die gesamte Aroma- oder Riechstoffmischung im Bereich von 0,00001 bis 40 Gew.-% liegt und die Einsatzmenge der Aroma- oder Riechstoffmischung (i+ii) bezogen auf die kosmetischen Produkte 2 bis 6 Gew.-% beträgt.

5. Produkte nach Anspruch 4 oder 5, wobei es sich um Wasch- und Reinigungsmittel, Hygiene- oder Pflegeprodukte handelt.

6. Produkte nach Anspruch 5, wobei es sich um Produkte für den Bereich der Körper- und/ oder Haarpflege, Shampoos, Weichspüler oder Waschpulver handelt.

7. Verwendung von Tetrahydrofuranderivaten der Formel (I) wie in Anspruch 1 oder 2 definiert,
(i) zum Vermitteln, Verstärken, Verbessern und/oder Modifizieren eines Geruchseindrucks, und/oder
(ii) zum Erhöhen der Substantivität oder Diffusität eines Riechstoffs oder einer Aroma- und/oder Riechstoffmischung.

8. Verwendung von Tetrahydrofuranderivaten der Formel (I) wie in Anspruch 1 oder 2 definiert,
(i) zum Vermitteln, Verstärken, Verbessern und/oder Modifizieren eines Geruchseindrucks in Richtung von Reinheit, Frische und/oder Natürlichkeit, und/oder
(ii) zum geruchlichen Abrunden einer Aroma- und/oder Riechstoffmischung oder eines kosmetischen oder Haushaltsproduktes.

9. Verwendung von Tetrahydrofuranderivaten der Formel (I) wie in Anspruch 1 oder 2 definiert, zum Vermitteln, Verstärken, Verbessern und/oder Modifizieren eines Geruchseindrucks in Richtung Volumen, Komplexität, Eleganz und/oder Natürlichkeit.

10. Verwendung von Tetrahydrofuranderivaten der Formel (I) wie in Anspruch 1 oder 2 definiert, in Aroma- und/oder Riechstoffmischungen zum Vermitteln eines pflegenderen, harmonischeren, hochwertigeren und/oder natürlicheren Geruchseindrucks in der Mischung im Vergleich zu einer Mischung, die keine Tetrahydrofuranderivate der Formel (I) wie in Anspruch 1 oder 2 definiert, enthalten.

11. Verwendung von Aroma- und/oder Riechstoffmischungen nach Anspruch 1 in kosmetischen Produkten oder Haushaltsprodukten
(i) zur Vermittlung, Verstärkung, Verbesserung und/oder Modifizierung eines Geruchseindrucks, und/oder
(ii) zur Erhöhung der Substantivität oder Diffusität eines Riechstoffs oder der Aroma- und/oder Riechstoffmischung und/oder
(iii) zur Vermittlung, Verstärkung, Verbesserung und/oder Modifizierung eines Geruchseindrucks in Richtung von Reinheit, Frische und/oder Natürlichkeit, und/oder
(iv) zur geruchlichen Abrundung der Aroma- und/oder Riechstoffmischung oder eines kosmetischen oder Haushaltsproduktes.

12. Tetrahydrofuranderivat der Formel (I), ausgewählt aus der Gruppe bestehend aus:
Tetrahydrofuran-2-carbonsäure-2-methylallylester (Verbindung 6) oder
Tetrahydrofuran-2-carbonsäure-isobutylester (Verbindung 7).

## Claims

1. Flavour and/or fragrance compositions, encompassing
(i) one or more tetrahydrofuran derivatives of formula (I) 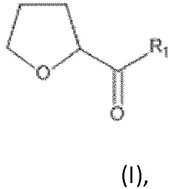
wherein R1 represents hydrogen, C1-C3 alkyl or a O-R2 residue,
wherein R2 represents a branched or linear C1-C5 alkyl group or a branched or linear C2-C6 alkenyl group, a substituted or unsubstituted cycloalkyl group, a substituted or unsubstituted (C1-C8) alkyl (C3-C7) cycloalkyl group, a substituted or unsubstituted (C2-C8) alkenyl (C3-C7) cycloalkyl group, a substituted or unsubstituted aryl group or a (C1-C8) alkylaryl group, a substituted or unsubstituted (C2-C8) alkenylaryl group,
whereby all stereoisomers of compound (I) are included, and
(ii) one or more additional flavour or fragrance compounds,
on condition that the total amount of tetrahydrofuran derivatives of formula (I) within said composition ranges from 0.00001 to 40 % by weight - calculated on the total flavour or fragrance composition.

2. Flavour and/or fragrance compositions according to Claim 1, wherein said tetrahydrofuran derivatives of formula (I) are selected from the group consisting of:
Compound 1: Tetrahydrofuran-2-carboxylic acid methyl ester
Compound 2: Tetrahydrofuran-2-carboxylic acid ethyl ester
Compound 3: Tetrahydrofuran-2-carboxylic acid propyl ester
Compound 4: Tetrahydrofuran-2-carboxylic acid allyl ester
Compound 5: Tetrahydrofuran-2-carboxylic acid -isopropyl ester
Compound 6: Tetrahydrofuran-2-carboxylic acid-2-methylallyl ester
Compound 7: Tetrahydrofuran-2-carboxylic acid -isobutyl ester
Compound 8: Tetrahydrofuran-2-carboxylic acid butyl ester
Compound 9: 1-(Tetrahydro-furan-2-yl)-ethanone.

3. Cosmetic products, comprising
(a) at least one compound of formula (I) as defined in Claim 1 or 2, or
(b) the flavour or fragrance composition according to Claim 1 or 2
on condition that the total amount of tetrahydrofuran derivatives of formula (I) within said composition ranges from 0.00001 to 40 % by weight - calculated on the total flavour or fragrance composition, and the working amount of said flavour or fragrance composition (i+ii) represents 2 to 6 % by weight - calculated on the cosmetic products.

4. Household products, comprising
(a) at least one compound of formula (I) as defined in Claim 1 or 2, or
(b) the flavour or fragrance composition according to Claim 1 or 2
on condition that the total amount of tetrahydrofuran derivatives of formula (I) within said composition ranges from 0.00001 to 40 % by weight - calculated on the total flavour or fragrance composition, and the working amount of said flavour or fragrance composition (i+ii) represents 2 to 6 % by weight - calculated on the cosmetic products.

5. Products according to Claim 4 or 5, representing washing, cleaning, hygiene or care compositions.

6. Products according to Claim 5 representing products for the segments of body and/or hair care, shampoos, softeners and detergents.

7. Use of tetrahydrofuran derivatives of formula (I) as defined in Claims 1 or 2,
(i) for providing, increasing, improving and/or modifying a flavour impression; and/or
(ii) for increasing substantivity and diffusion of a fragrance or a flavour and/or fragrance composition.

8. Use of tetrahydrofuran derivatives of formula (I) as defined in Claim 1 or 2,
(i) for providing, increasing, improving and/or modifying an olfactory impression in terms of purity, freshness and/or naturalness; and/or
(ii) for olfactory completeness of a flavour and/or fragrance composition of a cosmetic or household composition.

9. Use of tetrahydrofuran derivatives of formula (I) as defined in Claim 1 or 2, for providing, increasing, improving and/or modifying an olfactory impression in terms of volume, complexity, elegance and/or naturalness.

10. Use of tetrahydrofuran derivatives of formula (I) as defined in Claim 1 or 2, for providing a more nurturing, harmonic, high-quality and/or natural olfactory impression within a flavour and/or fragrance composition, when compared to a composition not comprising tetrahydrofuran derivatives as defined in Claim 1 or 2.

11. Use of flavour and/or fragrance compositions of Claim 1 in cosmetic or household products
(i) for providing, increasing, improving and/or modifying of an olfactory impression;
(ii) for increasing substantivity or diffusion of a fragrance or of the flavour and/or fragrance composition;
(iii) for providing, increasing, improving and/or modifying of an olfactory impression in terms of purity, freshness and/or naturalness; and/or
(iv) for olfactory completeness of a flavour and/or fragrance composition of a cosmetic or household composition.

12. Tetrahydrofuran derivative of formula (I) selected from the group consisting of Tetrahydrofuran-2-carboxylic acid-2-methylallyl ester (Compound 6) or Tetrahydrofuran-2-carboxylic acid isobutyl ester (Compound 7).

## Revendications

1. Mélanges d'arômes et/ou de parfums, comprenant :
(i) un ou plusieurs dérivés de tétrahydrofurane de formule (I) 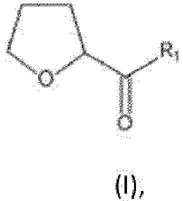
dans laquelle R1 représente un hydrogène, un alkyle en C1-C3 ou un radical O-R2,
R2 représentant un groupe alkyle en C1-C5 ramifié ou non ramifié, ou un groupe alcényle en C2-C6 ramifié ou non ramifié, un groupe cycloalkyle substitué ou non substitué, un groupe alkyle en (C₁-C₈)-cycloalkyle en (C₃-C₇) substitué ou non substitué, un groupe cycloalcényle substitué ou non substitué, un groupe alcényle en (C₂-C₈)-cycloalkyle en (C₃-C₇) substitué ou non substitué, un groupe aryle substitué ou non substitué, ou un groupe alkylaryle en (C₁-C₈) substitué ou non substitué, un groupe alcénylaryle en (C₂-C₈) substitué ou non substitué,
tous les stéréoisomères du composé (I) étant inclus, et
(ii) un ou plusieurs autres parfums ou arômes, à condition que la quantité totale de dérivés de tétrahydrofurane de formule (I) contenus, par rapport au mélange total d'arômes ou de parfums, se situe dans la plage allant de 0,00001 à 40 % en poids.

2. Mélanges d'arômes et/ou de parfums selon la revendication 1, dans lesquels les dérivés de tétrahydrofurane de formule (I) sont choisis dans le groupe constitué par :
composé 1 : ester méthylique de l'acide tétrahydrofurane-2-carboxylique
composé 2 : ester éthylique de l'acide tétrahydrofurane-2-carboxylique
composé 3 : ester propylique de l'acide tétrahydrofurane-2-carboxylique
composé 4 : ester allylique de l'acide tétrahydrofurane-2-carboxylique
composé 5 : ester isopropylique de l'acide tétrahydrofurane-2-carboxylique
composé 6 : ester 2-méthylallylique de l'acide tétrahydrofurane-2-carboxylique
composé 7 : ester isobutylique de l'acide tétrahydrofurane-2-carboxylique
composé 8 : ester butylique de l'acide tétrahydrofurane-2-carboxylique
composé 9 : 1-(tétrahydro-furan-2-yl)-éthanone.

3. Produits cosmétiques, comprenant :
(a) au moins un composé de formule (I), tel que défini dans la revendication 1 ou 2, ou
(b) des mélanges d'arômes ou de parfums selon la revendication 1 ou 2,
à condition que la quantité totale de dérivés de tétrahydrofurane contenus, par rapport au mélange total d'arômes ou de parfums, se situe dans la plage allant de 0,00001 à 40 % en poids, et que la quantité utilisée du mélange d'arômes ou de parfums (i+ii), par rapport aux produits cosmétiques, soit de 2 à 6 % en poids.

4. Produits ménagers, comprenant :
(a) au moins un composé de formule (I), tel que défini dans la revendication 1 ou 2, ou
(b) des mélanges d'arômes ou de parfums selon la revendication 1 ou 2,
à condition que la quantité totale de dérivés de tétrahydrofurane contenus, par rapport au mélange total d'arômes ou de parfums, se situe dans la plage allant de 0,00001 à 40 % en poids, et que la quantité utilisée du mélange d'arômes ou de parfums (i+ii), par rapport aux produits cosmétiques, soit de 2 à 6 % en poids.

5. Produits selon la revendication 4 ou 5, qui consistent en des détergents, des produits d'hygiène ou des produits de soin.

6. Produits selon la revendication 6, qui consistent en des produits pour le domaine du soin du corps et/ou des cheveux, des shampoings, des assouplissants ou des poudres de lavage.

7. Utilisation de dérivés de tétrahydrofurane de formule (I) tels que définis dans la revendication 1 ou 2,
(i) pour le développement, le renforcement, l'amélioration et/ou la modification d'une sensation olfactive et/ou
(ii) pour l'augmentation de la substantivité ou de la diffusivité d'un parfum ou d'un mélange d'arômes et/ou de parfums.

8. Utilisation de dérivés de tétrahydrofurane de formule (I) tels que définis dans la revendication 1 ou 2,
(i) pour le développement, le renforcement, l'amélioration et/ou la modification d'une sensation olfactive dans la direction de la pureté, de la fraîcheur et/ou du naturel, et/ou
(ii) pour l'arrondissement olfactif d'un mélange d'arômes et/ou de parfums ou d'un produit cosmétique ou ménager.

9. Utilisation de dérivés de tétrahydrofurane de formule (I) tels que définis dans la revendication 1 ou 2, pour le développement, le renforcement, l'amélioration et/ou la modification d'une sensation olfactive dans la direction du volume, de la complexité, de l'élégance et/ou du naturel.

10. Utilisation de dérivés de tétrahydrofurane de formule (I) tels que définis dans la revendication 1 ou 2 dans des mélanges d'arômes et/ou de parfums pour le développement d'une sensation olfactive soignante, harmonisante, de grande qualité et/ou naturelle dans le mélange en comparaison d'un mélange qui ne contient pas de dérivés de tétrahydrofurane de formule (I) tels que définis dans la revendication 1 ou 2.

11. Utilisation de mélanges d'arômes et/ou de parfums selon la revendication 1 dans des produits cosmétiques ou des produits ménagers,
(i) pour le développement, le renforcement, l'amélioration et/ou la modification d'une sensation olfactive et/ou
(ii) pour l'augmentation de la substantivité ou de la diffusivité d'un parfum ou du mélange d'arômes et/ou de parfums, et/ou
(iii) pour le développement, le renforcement, l'amélioration et/ou la modification d'une sensation olfactive dans la direction de la pureté, de la fraîcheur et/ou du naturel, et/ou
(ii) pour l'arrondissement olfactif du mélange d'arômes et/ou de parfums ou d'un produit cosmétique ou ménager.

12. Dérivé de tétrahydrofurane de formule (I), choisi dans le groupe constitué par :
l'ester 2-méthylallylique de l'acide tétrahydrofurane-2-carboxylique (composé 6) ou
l'ester isobutylique de l'acide tétrahydrofurane-2-carboxylique (composé 7).
